# EUROPEAN PATENT APPLICATION

(11) **EP 2 515 615 A1**
(43) Date of publication of application: **24.10.2012**
(21) Application number: 10837576.7
(22) Date of filing: 13.12.2010
(51) Int. Cl.: H05B 33/10, C09K 11/06, H01L 51/50, C07D 215/30, C07D 307/91, C07D 401/10

(54) **METHOD FOR MANUFACTURING ORGANIC ELECTROLUMINESCENT ELEMENT, ORGANIC ELECTROLUMINESCENT ELEMENT, DISPLAY DEVICE AND ILLUMINATING DEVICE**

(30) Priority: 15.12.2009 JP 2009284384; 27.01.2010 JP 2010016030
(71) Applicant: Mitsubishi Chemical Corporation, Chiyoda-ku Tokyo 100-8251 (JP)
(72) Inventor: TSURUTANI Yasuyuki, Yokohama-shi Kanagawa 227-8502 (JP); SATO Hideki, Yokohama-shi Kanagawa 227-8502 (JP); IMADA Ichiro, Yokohama-shi Kanagawa 227-8502 (JP)
(74) Representative: HOFFMANN EITLE
(86) International application number: PCT/JP2010/072407
(87) International publication number: WO 2011/074550

(57) **Abstract**

A task/object of the present invention is to produce an organic electroluminescence element having high driving stability and long drive life at a low cost by forming a luminescent layer in a specific environment by using a luminescent layer-forming composition containing a compound having a specific structure and a solvent. The invention relates to a method for manufacturing an organic electroluminescence element having a luminescent layer between a first electrode and a second electrode formed to face said first electrode, the method comprising, as a luminescent layer-forming step, a step of forming a coating film by a wet film-forming method in an environment with an oxygen concentration of 18 to 22 vol% by using a luminescent layer-forming composition containing: a compound having a molecular weight of 400 or more and represented by the following formula (1); and a solvent (wherein each of Ar³¹ and Ar³² independently represents an aromatic hydrocarbon ring group or aromatic heterocyclic group having a substituent, provided that said compound represented by formula (1) does not have a partial structure derived from a nitrogen atom-containing heterocyclic ring, and the anthracene ring in formula (1) may further have an optional substituent):

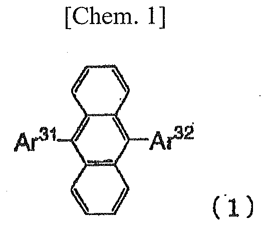

## Description

### TECHNICAL FIELD

The present invention relates to a method for manufacturing an organic electroluminescence element with high luminous efficiency and high driving stability, an organic electroluminescence element manufactured by the manufacturing method, and a display device and a lighting device each having the organic electroluminescence element.

### BACKGROUND ART

Recently, development of an electroluminescence element using an organic thin film (organic electroluminescence element) is proceeding. The method for forming an organic thin film includes a vacuum deposition method and a wet film-forming method. Of these, the wet film-forming method is advantageous in that, for example, a vacuum process is not required, large-area deposition is facilitated, and a plurality of materials having various functions can be easily mixed and incorporated into one layer (coating solution).

As the material of a luminescent layer formed by a wet film-forming method, a polymer material such as poly(p-phenylene vinylene) derivative and polyfluorene derivative is predominantly used, but the polymer material has the following problems:
● the polymerization degree or molecular weight distribution of the polymer material is difficult to control,
● deterioration due to a residue at the molecular terminal as an active site of the polymer material occurs during continuous driving, and
● the material itself can be hardly purified to high purity and contains impurities.
   Because of these problems, at present, the organic electroluminescence element by a wet film-forming method is poor in the driving stability as compared with the organic electroluminescence element by a vacuum deposition method and fails in reaching a practical level with partial exception.

As an attempt to solve the problems above, Patent Document 1 describes an organic electroluminescence element using an organic thin film formed by mixing a plurality of low molecular materials (charge transport material, luminescent material) and wet-depositing the mixture.

Also, it is stated in Patent Document 2 that when an anthracene compound is used as a charge transport material, an element with high color purity and high driving stability is obtained, and Patent Document 3 describes a technique where an ink composed of an anthracene derivative and a condensed ring substituted with a diarylamine is used for the manufacture of an organic electroluminescence element.

On the other hand, an organic layer constituting an organic electroluminescence element and being formed by a wet film-forming method, particularly, a luminescent functional material or the like contained in such an organic layer, is sometimes deteriorated (damaged) by the contact with moisture, oxygen, ozone or the like in air, and there is also a possibility that attachment/mixing of an impurity is generated, for example, by adsorption of a component in the atmosphere to the film surface (Patent Documents 4 and 5). Accordingly, in conventional techniques, when producing an organic electroluminescence element by a wet film-forming method, it is recommended to perform the production in an inert gas such as dry nitrogen, as described, for example, in Patent Documents 6 and 7.

### RELATED ART

### PATENT DOCUMENT

Patent Document 1: JP-A-11-273859 (the term "JP-A" as used herein means an "unexamined published Japanese patent application")
Patent Document 2: U.S. Patent Application Publication No. 2009/200929
Patent Document 3: U.S. Patent Application Publication No. 2008/001123
Patent Document 4: JP-A-2007-273093
Patent Document 5: JP-A-2007-207762
Patent Document 6: JP-A-2002-170676
Patent Document 7: JP-A-2002-170672

### SUMMARY OF THE INVENTION

### PROBLEMS THAT THE INVENTION IS TO SOLVE

However, the organic electroluminescence elements described in Patent Documents 1 to 3 have a problem that the drive life is short.
Also, the methods described in Patent Documents 6 and 7 have a problem in the production cost.

A task/object of the present invention is to produce an organic electroluminescence element having high driving stability and long drive life at a low cost by forming a luminescent layer in a specific environment from a luminescent layer-forming composition containing a compound having a specific structure and a solvent.

### MEANS FOR SOLVING THE PROBLEMS

As a result of intensive studies, the present inventors have found that at the time of forming a luminescent layer by a wet film-forming method, when a luminescent layer-forming composition containing an anthracene compound having a specific structure is used and the environment for forming a coating film is exposed to specific conditions, the above-described object can be attained. The present invention has been accomplished based on this finding.
That is, the gist of the present invention resides in the following (1) to (10). (1) A method for manufacturing an organic electroluminescence element having a luminescent layer between a first electrode and a second electrode formed to face said first electrode, the method comprising,
as a luminescent layer-forming step, forming a coating film by a wet film-forming method in an environment with an oxygen concentration of 18 to 22 vol% by using a luminescent layer-forming composition containing: a compound having a molecular weight of 400 or more and represented by the following formula (1); and a solvent:

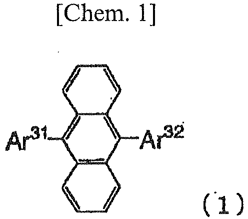

(wherein each of Ar³¹ and Ar³² independently represents an aromatic hydrocarbon ring group or aromatic heterocyclic group having a substituent, provided that said compound represented by formula (1) does not have a partial structure derived from a nitrogen atom-containing heterocyclic ring, and the anthracene ring in formula (1) may further have an optional substituent).
(2) The method of manufacturing an organic electroluminescence element as described in the item (1) above, which further comprises heating said coating film in an inert gas.
(3) The method of manufacturing an organic electroluminescence element as described in the item (1) or (2) above, wherein the molecular weight of said compound represented by formula (1) is 10,000 or less.
(4) The method of manufacturing an organic electroluminescence element as described in any one of the items (1) to (3) above, wherein said compound represented by formula (1) is a charge transport material.
(5) The method of manufacturing an organic electroluminescence element as described in any one of the items (1) to (4) above, wherein said luminescent layer-forming composition further contains a luminescent material and said luminescent material contains a condensed aromatic hydrocarbon ring having a nuclear carbon number of 5 to 40, which may be substituted.
(6) The method of manufacturing an organic electroluminescence element as described in the item (5) above, wherein said luminescent material contains at least one compound selected from the group consisting of naphthalene, perylene, pyrene, chrysene, anthracene, coumarin, p-bis(2-phenylethenyl)benzene, a styrylamine compound represented by the following formula (2), and an arylamine compound represented by the following formula (3):

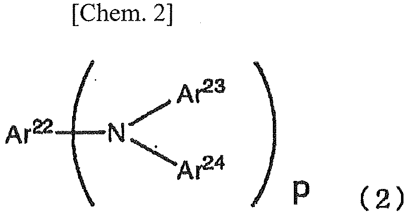

(wherein Ar²² represents a group derived from an aromatic hydrocarbon ring or an aromatic heterocyclic ring, or a stilbene-derived group, each of which may be substituted,
each of Ar²³ and Ar²⁴ independently represents a hydrogen atom, a styryl group which may be substituted, or an aromatic hydrocarbon ring group having a carbon number of 6 to 20, which may be substituted, and
p represents an integer of 1 to 4,
provided that when Ar²² is not a stilbene-derived group and at the same time, the group derived from an aromatic hydrocarbon ring or an aromatic heterocyclic ring is not substituted with a styryl group, at least one of Ar²³ and Ar²⁴ is a styryl group which may be substituted);

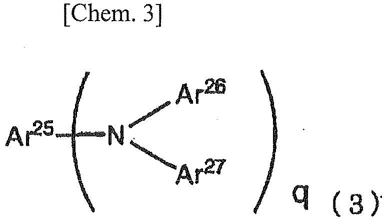

(wherein Ar²⁵ represents an aromatic hydrocarbon ring-derived group having a nuclear carbon number of 10 to 40, which may be substituted, or an aromatic heterocyclic ring-derived group which may be substituted,
each of Ar²⁶ and Ar²⁷ independently represents an aromatic hydrocarbon ring-derived group having a nuclear carbon number of 5 to 40, which may be substituted, or an aromatic heterocyclic ring-derived group which may be substituted, and
q represents an integer of 1 to 4).
(7) The method of manufacturing an organic electroluminescence element as described in the item (5) or (6) above, wherein the content of said compound represented by formula (1) in said luminescent layer-forming composition is, on the weight basis, 2 times or more the entire amount of said luminescent material.
(8) An organic electroluminescence element manufactured by the manufacturing method as described in any one of the items (1) to (7) above.
(9) A display device comprising the organic electroluminescence element as described in the item (8) above.
(10) A lighting device comprising the organic electroluminescence element as described in the item (8) above.

### ADVANTAGE OF THE INVENTION

According to the present invention, a coating film is formed by a wet film-forming method in an environment with an oxygen concentration of 18 to 22 vol% by using a luminescent layer-forming composition containing an anthracene compound having a specific structure, whereby an organic electroluminescence element having high driving stability and long drive life can be manufactured.
Furthermore, in the present invention, the environment for forming a luminescent layer by a wet film-forming method need not be a nitrogen atmosphere or an environment with a low oxygen concentration, so that an organic electroluminescence element can be manufactured at a low production cost.
Accordingly, the organic electroluminescence element manufactured by the manufacturing method of the present invention is suited for a display device or a lighting device and, specifically, is thought to be applicable to flat panels/displays (for example, an office automation (OA) computer display and a thin television), vehicle-mounted display elements, cellular phone displays, light sources taking advantage of the feature of a surface light emitter (for example, a light source of copiers and a backlight source of liquid-crystal displays or instruments), display boards, and marker lights, and its technical value is very high.

### BRIEF DESCRIPTION OF THE DRAWINGS

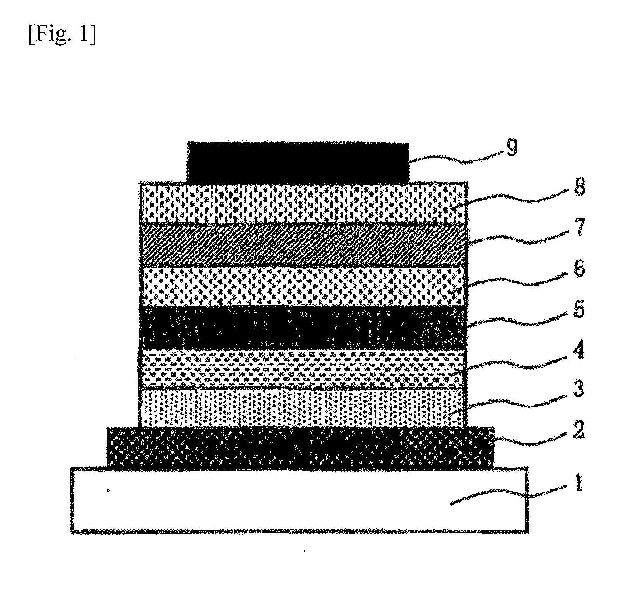

[Fig. 1] A cross-sectional view schematically showing one example of the structure of the organic electroluminescence element of the present invention.

### BEST MODE FOR CARRYING OUT THE INVENTION

The mode for carrying out the present invention is described in detail below, but the following descriptions of constituent elements are absolutely a mere example (representative example) of the embodiment of the present invention. The present invention is not limited to these contents as long as its gist is observed.
The "wt%", "ppm by weight" and "parts by weight" have the same meanings as "mass%", "ppm by mass" and "parts by mass", respectively.

### <Manufacturing Method of Organic Electroluminescence Element>

The manufacturing method of the present invention is a method for manufacturing an organic electroluminescence element having a luminescent layer between a first electrode and a second electrode formed to face the first electrode, wherein in forming the luminescent layer, a coating film is formed by a wet film-forming method in an environment with an oxygen concentration of 18 to 22 vol% by using a luminescent layer-forming composition containing a compound having a molecular weight of 400 or more represented by the following formula (1) and a solvent. From the standpoint of enhancing the luminous efficiency, drive life and driving stability of the organic electroluminescence element, the manufacturing method of the present invention preferably comprises a step of further heating the coating film in an inert gas.

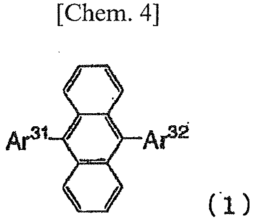

(wherein each of Ar³¹ and Ar³² independently represents an aromatic hydrocarbon ring group or aromatic heterocyclic group having a substituent, provided that the compound represented by formula (1) does not have a partial structure derived from a nitrogen atom-containing heterocyclic ring, and
the anthracene ring in formula (1) may further have an optional substituent).

### [Luminescent layer]

The luminescent layer in the present invention is formed by a wet film-forming method in an environment with an oxygen concentration of 18 to 22 vol% by using a luminescent layer-forming composition containing a compound having a molecular weight of 400 or more represented by formula (1) and a solvent. Also, as described above, the film deposition is preferably performed by further heating the formed coating film of the luminescent layer in an inert gas.

### [Luminescent layer-Forming Composition]

The luminescent layer-forming composition for use in the present invention must contain the below-described compound having a molecular weight of 400 or more represented by formula (1) (hereinafter, sometimes referred to as a "specific low-molecular compound") and a solvent. If desired, the composition preferably further contains the later-described luminescent material (hereinafter, sometimes referred to as a "dopant").

### (Compound having a molecular weight of 400 or more represented by formula (1))

The luminescent layer-forming composition for use in the present invention must contain a compound having a molecular weight of 400 or more represented by the following formula (1):

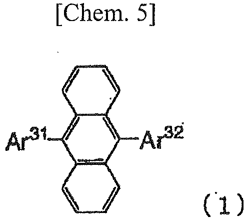

(wherein each of Ar³¹ and Ar³² independently represents an aromatic hydrocarbon ring group or aromatic heterocyclic group having a substituent, provided that the compound represented by formula (1) does not have a partial structure derived from a nitrogen atom-containing heterocyclic ring, and
the anthracene ring in formula (1) may further have an optional substituent).

Each of Ar³¹ and Ar³² independently represents an aromatic hydrocarbon ring group or aromatic heterocyclic group having a substituent.
Examples of the aromatic hydrocarbon ring group of Ar³¹ and Ar³² include a group derived from a benzene ring or a condensed ring formed by fusing from 2 to 5 benzene rings, such as naphthalene ring, azulene ring, phenanthrene ring, anthracene ring, pyrene ring, chrysene ring, naphthacene ring, benzophenanthrene ring and triphenylene ring.
Examples of the aromatic heterocyclic group of Ar³¹ and Ar³² include a group derived from a furan ring, a dibenzofuran ring, a benzofuran ring, a thiophene ring, a benzothiophene ring, a thienothiophene ring, a furofuran ring or a thienofuran ring.

In the present invention, for example, in view of the thermal stability and solubility for an organic solvent of the specific low-molecular compound as well as the charge transport efficiency and injection performance by the specific low-molecular compound, each of Ar³¹ and Ar³² is independently preferably an aromatic hydrocarbon ring group having a substituent, more preferably a group derived from a benzene ring, a naphthalene ring, an anthracene ring or a pyrene ring.

In the specific low-molecular compound for use in the present invention, for the reason that by increasing the molecular weight, thermal stability and solubility for an organic solvent can be elevated and crystallization in the film during deposition can be prevented, each of the aromatic hydrocarbon group and the aromatic heterocyclic group of Ar³¹ and Ar³² must further have a substituent.

Examples of the substituent substituted on Ar³¹ and Ar³² include an alkyl group, an aromatic hydrocarbon ring group, an aromatic heterocyclic group, an alkoxy group, a (hetero)aryloxy group, an alkylthio group, a (hetero)arylthio group, a cyano group, a dialkylamino group, an alkylarylamino group, and a diarylamino group. Among these, an alkyl group and an aromatic hydrocarbon ring group are preferred in view of stability of the compound, and an aromatic hydrocarbon ring group is more preferred. Incidentally, the term "(hetero)aryl" as used above indicates both an aryl and a heteroaryl.

The alkyl group is preferably an alkyl group having a carbon number of 1 to 20, such as methyl group, ethyl group, propyl group, iso-propyl group, butyl group, iso-butyl group, sec-butyl group, tert-butyl group, hexyl group, octyl group, cyclohexyl group, decyl group and octadecyl group, more preferably an alkyl group having a carbon number of 1 to 4, such as methyl group, ethyl group, iso-propyl group, sec-butyl group and tert-butyl group, and in view of availability and low cost of the raw material, still more preferably a methyl group or an ethyl group. On the other hand, an iso-butyl group, a sec-butyl group and a tert-butyl group are also more preferred because of their high solubility in a nonpolar solvent.

The aromatic hydrocarbon ring group is preferably a group having a carbon number of 6 to 25, for example, derived from a benzene ring, a naphthalene ring, an azulene ring, an anthracene ring, a phenanthrene ring, a perylene ring, a tetracene ring, a pyrene ring, a benzopyrene ring, a chrysene ring, a triphenylene ring or a fluoranthene ring, more preferably a group derived from a benzene ring, a naphthalene ring, an anthracene ring or a phenanthrene ring.

The aromatic heterocyclic group is preferably a group derived from a furan ring, a dibenzofuran ring, a benzofuran ring, a thiophene ring, a benzothiophene ring, a thienothiophene ring, a furofuran ring or a thienofuran ring, more preferably a group derived from a furan ring, a benzofuran ring, a thiophene ring, a dibenzofuran ring or a benzothiophene ring.

The alkoxy group is preferably an alkoxy group having a carbon number of 1 to 20, such as methoxy group, ethoxy group, isopropyloxy group, cyclohexyloxy group and octadecyloxy group, more preferably a methoxy group, an ethoxy group or an isopropyloxy group.

The (hetero)aryloxy group is preferably a (hetero)aryloxy group having a carbon number of 3 to 20, such as phenoxy group, 1-naphthyloxy group, 9-anthranyloxy group and 2-thienyloxy group, more preferably a phenoxy group or a 1-naphthyloxy group.

The alkylthio group is preferably an alkylthio group having a carbon number of 1 to 20, such as methylthio group, ethylthio group, isopropylthio group and cyclohexylthio group, more preferably a methylthio group, an ethylthio group, or an isopropylthio group.

The (hetero)arylthio group is preferably a (hetero)arylthio group having a carbon number of 3 to 20, such as phenylthio group, 1-naphthylthio group, 2-naphthylthio group, 9-anthranylthio group and 2-thienylthio group, more preferably a phenylthio group, a 1-naphthylthio group, or a 2-naphthylthio group.

The dialkylamino group is preferably a dialkylamino group having a carbon number of 2 to 29, such as dimethylamino group, diethylamino group, diisopropylamino group and methylethylamino group, more preferably a dimethylamino group, a diethylamino group, or a diisopropylamino group.

The alkylarylamino group is preferably an alkylarylamino group having a carbon number of 7 to 30, such as methylphenylamino group.

The diarylamino group is preferably a diarylamino group having a carbon number of 12 to 30, such as diphenylamino group and phenylnaphthylamino group.

These substituents may further have a substituent, and examples of the substituent which may be substituted on these substituents include the above-described alkyl group, aryl group, alkoxy group and diarylamino group.

The anthracene ring in formula (1) may further have an optional substituent as long as the effects of the present invention are not impaired. Examples of the optional substituent include the same groups as those for the substituent on the aromatic hydrocarbon ring group and aromatic heterocyclic group in Ar³¹ and Ar³², but from the standpoint of enhancing the thermal stability and solubility for an organic solvent of the specific low-molecular compound and the driving stability of an organic electroluminescence element using the specific low-molecular compound, the anthracene ring is preferably unsubstituted.

In the present invention, the molecular weight of the specific low-molecular compound is usually 10,000 or less, preferably 5,000 or less, more preferably 4,000 or less, still more preferably 3,000 or less, and is usually 400 or more, preferably 450 or more, more preferably 480 or more, still more preferably 500 or more.

When the molecular weight is in the range above, the compound exhibits good heat resistance and therefore, is less likely to cause gas generation, and the film quality of a film formed is also good. In addition, this is advantageous in that, for example, purification of the compound is facilitated and in turn, the purity is easily elevated.

Incidentally, the specific low-molecular compound of the present invention does not have, in its chemical structure, a partial structure derived from a nitrogen atom-containing heterocyclic ring. This is because a compound having a partial structure derived from a nitrogen atom-containing heterocyclic ring exhibits excessively high electron transport/injection performance and it is considered that even when coated in the atmosphere, the later-described luminescence/recombination site is not formed at the side of the luminescent layer nearer the cathode and the effects of the present invention are not prominently brought out.

On the other hand, the specific low-molecular compound preferably has a pyrene ring, a naphthalene ring or the like in the chemical structure. By virtue of having such a ring, the oxidation-reduction resistance of the compound is enhanced. Also, when a composition containing such a compound is coated in an inert gas such as nitrogen, a luminescence/recombination site is liable to be generated nearer the anode, but thanks to coating in the atmosphere, oxygen is contained in the coating film and for the later-described reason, a luminescence/recombination site is formed at the side of the luminescent layer nearer the cathode. In turn, the effects of the present invention are considered to be brought out more prominently.

In the present invention, the specific low-molecular compound has a very excellent charge transporting performance and therefore, is preferably used as a charge transport material (hereinafter, sometimes referred to as a "host") when forming a luminescent layer.

Preferred examples of the specific low-molecular compound are specifically illustrated below, but the present invention is not limited thereto.

### (Specific Examples of Charge Transport Material)

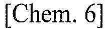

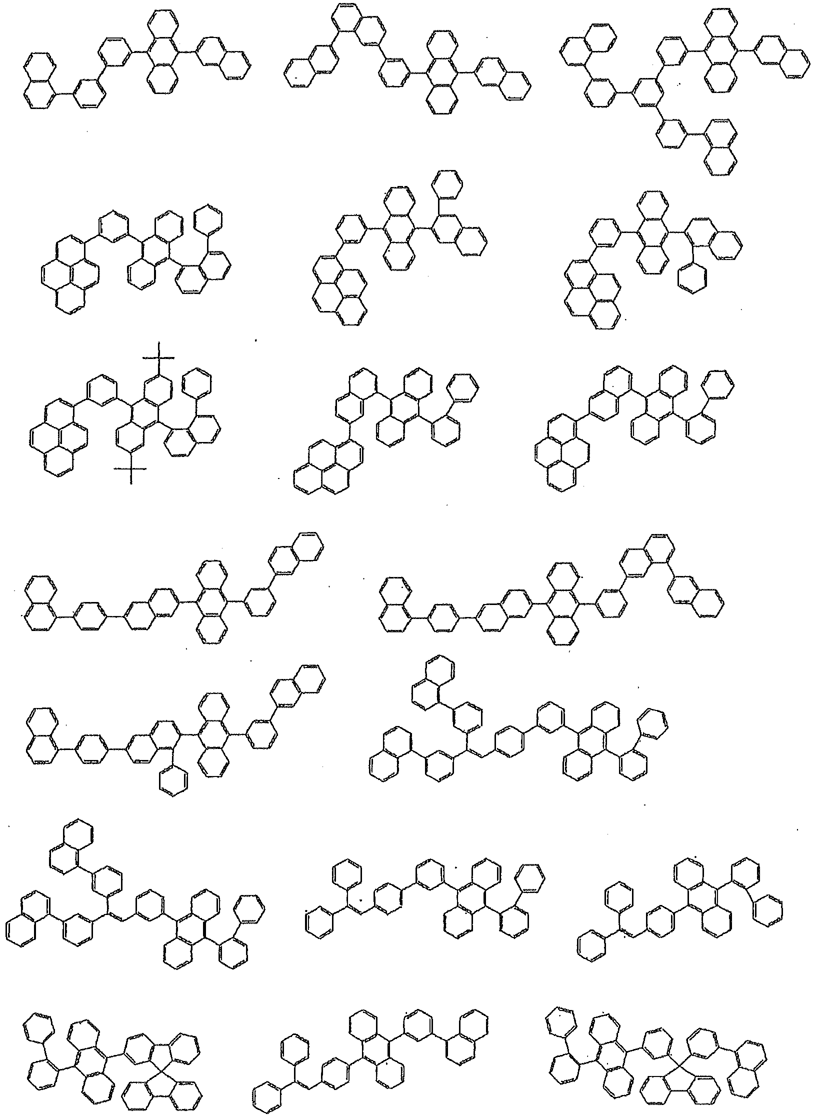

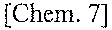

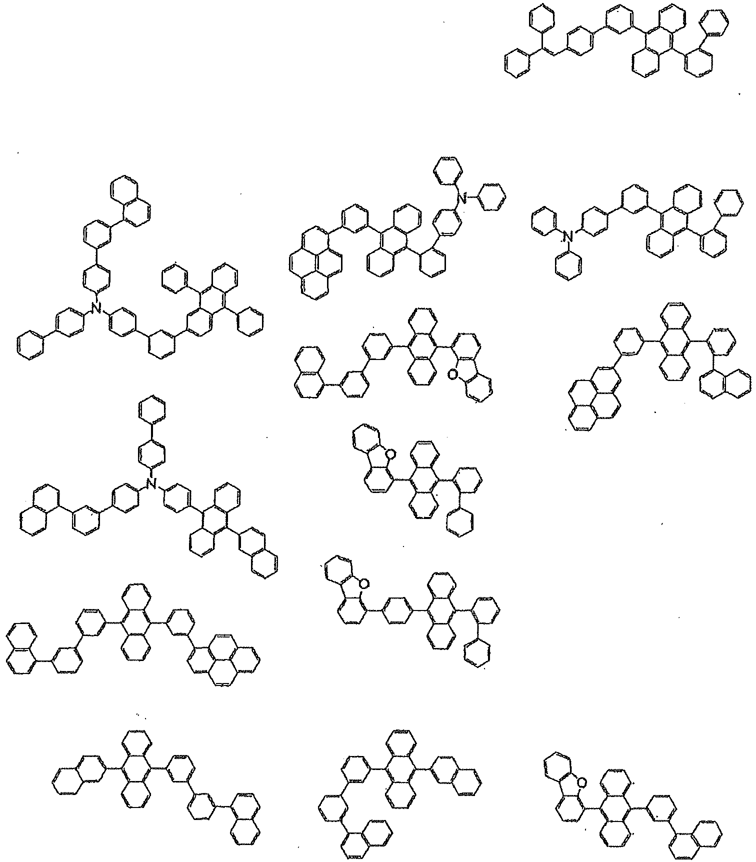

### (Solvent)

The luminescent layer-forming composition for use in the present invention must contain a solvent.
Here, the solvent as used in the present invention is a compound which is liquid in an atmosphere of 20°C and 1 atom and can dissolve the host or the later-described luminescent material contained in the luminescent layer-forming composition for use in the present invention.

The solvent is not particularly limited as long as it is a polar or apolar solvent generally available on the market, but above all, a substituted or unsubstituted aromatic hydrocarbon-based solvent such as benzene, toluene, xylene, mesitylene, cyclohexylbenzene, chlorobenzene and dichlorobenzene, an aromatic ether-based solvent such as anisole, benzoic acid ester and diphenyl ether, an aromatic ester-based solvent, a chain or cyclic alkane-based solvent such as hexane, heptane and cyclohexane, a carboxylic acid ester-based solvent such as ethyl acetate, a carbonyl-containing solvent such as acetone and cyclohexanone, water, an alcohol, a cyclic ether and the like are preferred, an aromatic hydrocarbon-based solvent is more preferred, and benzene, toluene, mesitylene and cyclohexylbenzene are still more preferred. Above all, a low-boiling-point solvent such as benzene, toluene, xylene, and mesitylene is preferred.

The low-boiling-point solvent as used herein is a solvent having a boiling point of usually 50°C or more, preferably 70°C or more, and usually 200°C or less, preferably 180°C or less. The boiling point in this range is preferred, because the coating film is more uniformly obtained.

The luminescent layer-forming composition for use in the present invention may contain one kind of a solvent or two or more kinds of solvents, but it is preferred to contain usually one or more kinds, preferably two or more kinds, and usually ten or less kinds, preferably eight or less kinds, more preferably six or less kinds, of solvents.

In the case where the luminescent layer-forming composition for use in the present invention is used to form a luminescent layer of the later-described organic electroluminescent element of the present invention, the content of the solvent in the luminescent layer-forming composition is arbitrary as long as the effects of the present invention are not seriously impaired, but the content is usually 50 wt% or more and is usually 99.99 wt% or less. In the case of using a mixture of two or more kinds of solvents, it may suffice if the total of these solvents satisfies the range above.

Also, the entire concentration of solid contents such as charge transport material and the later-described luminescent material is usually 0.01 wt% or more and is usually 70 wt% or less. If this concentration is too large, thickness unevenness may be caused, whereas if it is too small, a defect may be produced in the film.

### (Luminescent Material)

In view of emission wavelength, luminous efficiency, driving stability and the like of the organic electroluminescence element fabricated, the luminescent layer-forming composition for use in the present invention preferably further contains a luminescent material.
The luminescent material may be a fluorescent material or a phosphorescent material, and an arbitrary known material may be applied, but for the following reasons, use of a fluorescent material is preferred in the present invention.
In principle, the fluorescent material is lower in the luminous efficiency of the organic electroluminescence element than the phosphorescent material, but the energy gap in the singlet excited state is smaller than that of a phosphorescent material at the same emission wavelength and moreover, the exciton life is very short of the nanosecond order, as a result, the load imposed on the luminescent material is small and the drive life of the element is liable to be prolonged.

In the present invention, the molecular weight of the compound used as the dopant is arbitrary as long as the effects of the present invention are not seriously impaired, but the molecular weight is usually 10,000 or less, preferably 5,000 or less, more preferably 4,000 or less, still more preferably 3,000 or less, and is usually 100 or more, preferably 200 or more, more preferably 300 or more, still more preferably 400 or more.
The molecular weight in the range above is preferred because the compound exhibits good heat resistance and is less likely to cause gas generation and the film quality of a film formed is also good. Furthermore, for example, purification is facilitated and in turn, the purity is easily elevated.

In the present invention, for the purpose of enhancing the solubility in a solvent, it is preferred to reduce the molecular symmetry or rigidity of the dopant molecule or introduce a lipophilic substituent such as alkyl group.

Examples of the fluorescent material giving green luminescence (green fluorescent dye) include a quinacridone derivative, a coumarin derivative, and an aluminum complex such as Al(C₉H₆NO)₃.

Examples of the fluorescent material giving yellow luminescence (yellow fluorescent dye) include rubrene and perimidone derivatives.

Examples of the fluorescent material giving red luminescence (red fluorescent dye) include a DCM (4-(dicyanomethylene)-2-methyl-6-(p-dimethylaminostyryl)-4H-pyran)-based compound, a benzopyran derivative, a Rhodamine derivative, a benzothioxanthene derivative, and an azabenzothioxanthene derivative.

Examples of the fluorescent material giving blue luminescence (blue fluorescent dye) include a substituted or unsubstituted condensed aromatic hydrocarbon ring having a nuclear carbon number of 5 to 40. Above all, for example, naphthalene, perylene, pyrene, chrysene, anthracene, coumarin, p-bis(2-phenylethenyl)benzene, a styrylamine compound represented by the following formula (2), and an arylamine compound represented by the following formula (3) are preferred in view of emission wavelength, luminous efficiency, driving stability and the like of the organic electroluminescence element fabricated.

Among the fluorescent materials (blue fluorescent dyes), from the standpoint that high color purity of blue is realized and high efficiency and long life can be achieved by efficiently trapping a hole in the luminescent layer, a styrylamine compound represented by the following formula (2) and an arylamine compound represented by the following formula (3) are more preferred.

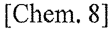

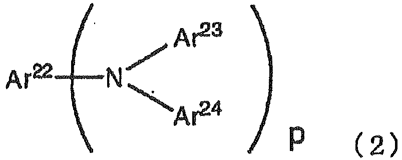

(wherein Ar²² represents a group derived from an aromatic hydrocarbon ring or an aromatic heterocyclic ring, or a stilbene-derived group, each of which may be substituted,
each of Ar²³ and Ar²⁴ independently represents a hydrogen atom, a styryl group which may be substituted, or an aromatic hydrocarbon ring group having a carbon number of 6 to 20, which may be substituted, and
p represents an integer of 1 to 4,
provided that when Ar²² is not a stilbene-derived group and at the same time, the group derived from an aromatic hydrocarbon ring or an aromatic heterocyclic ring is not substituted with a styryl group, at least either one of Ar²³ and Ar²⁴ is a styryl group which may be substituted).

Examples of the aromatic hydrocarbon ring group-derived group of Ar²² include a group derived from a benzene ring or a condensed ring formed by fusing from 2 to 5 benzene rings, such as naphthalene ring, azulene ring, phenanthrene ring, anthracene ring, pyrene ring, chrysene ring, naphthacene ring, benzophenanthrene ring and triphenylene ring.

Examples of the aromatic heterocyclic group of Ar²² include a group derived from a furan ring, a dibenzofuran ring, a benzofuran ring, a thiophene ring, a benzothiophene ring, a thienothiophene ring, a furofuran ring or a thienofuran ring.

Examples of the substituent which may be substituted on Ar²², Ar²³ and Ar²⁴ include the same groups as those of the substituent substituted on Ar³¹ and Ar³² in formula (1).
Incidentally, examples of the aromatic hydrocarbon ring group having a carbon number of 6 to 20 of Ar²³ and Ar²⁴ include a phenyl group, a naphthyl group, an anthranyl group, and a phenanthryl group. Examples of the substituent which may be further substituted on the aromatic hydrocarbon ring group having a carbon number of 6 to 20 also include the same groups as those of the substituent substituted on Ar³¹ and Ar³² in formula (1).

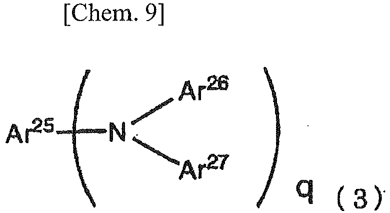

(wherein Ar²⁵ represents an aromatic hydrocarbon ring-derived group having a nuclear carbon number of 10 to 40, which may be substituted, or an aromatic heterocyclic ring-derived group which may be substituted,
each of Ar²⁶ and Ar²⁷ independently represents an aromatic hydrocarbon ring-derived group having a nuclear carbon number of 5 to 40, which may be substituted, or an aromatic heterocyclic ring-derived group which may be substituted, and
q represents an integer of 1 to 4).

Examples of the aromatic hydrocarbon ring-derived group having a nuclear carbon number of 10 to 40 include a group derived from a naphthalene ring, an anthracene ring, a pyrene ring, a chrysene ring, a fluorene ring or a fluoranthene ring.
Examples of the aromatic hydrocarbon ring-derived group having a nuclear carbon number of 5 to 40 include a group derived from a benzene ring, a naphthalene ring, an anthracene ring, a pyrene ring, a chrysene ring, a fluorene ring or a fluoranthene ring.
Examples of the aromatic heterocyclic ring-derived group include a group derived from an imidazole ring, a carbazole ring, a dibenzofuran ring or a dibenzothiophene ring.

Preferred examples of the substituent on the aromatic hydrocarbon ring-derived group having a nuclear carbon number of 10 to 40, the aromatic hydrocarbon ring-derived group having a nuclear carbon number of 5 to 40, and the aromatic heterocyclic ring-derived group include an alkyl group having a carbon number of 1 to 6 (e.g. ethyl, methyl, i-propyl, n-propyl, s-butyl, tert-butyl, pentyl, hexyl, cyclopentyl, cyclohexyl), an alkoxy group having a carbon number of 1 to 6 (e.g. ethoxy, methoxy, i-propoxy, n-propoxy, s-butoxy, tert-butoxy, pentoxy, hexyloxy, cyclopentoxy, cyclohexyloxy), an aryl group having a nuclear carbon number of 5 to 40, an amino group substituted with an aryl group having a nuclear carbon number of 5 to 40, an ester group containing an aryl group having a nuclear carbon number of 5 to 40, an ester group containing an alkyl group having a carbon number of 1 to 6, a cyano group, a nitro group, and a halogen atom.

Only any one of the above-described luminescent materials may be used, or two or more thereof may be used in arbitrary combination at an arbitrary ratio.

In the present invention, the proportion of the luminescent material in the luminescent layer-forming composition is arbitrary as long as the effects of the present invention are not seriously impaired, but the proportion is usually from 0.05 to 20 wt%, preferably from 0.1 to 10 wt%, based on the entire solid content. Also, the content of the compound represented by formula (1) is usually, on the weight basis, 2 times or more (200 wt% or more), preferably 3 times or more (300 wt% or more), more preferably 5 times or more (500 wt% or more), the entire amount of the luminescent material. If the proportion of the luminescent material is too small, luminescence unevenness may be caused, whereas if it is excessive large, the luminous efficiency may decrease. In the case of using two or more luminescent materials in combination, the total content thereof is adjusted to fall in the range above.

Specific preferred examples of the dopant are illustrated below, but the present invention is not limited thereto.

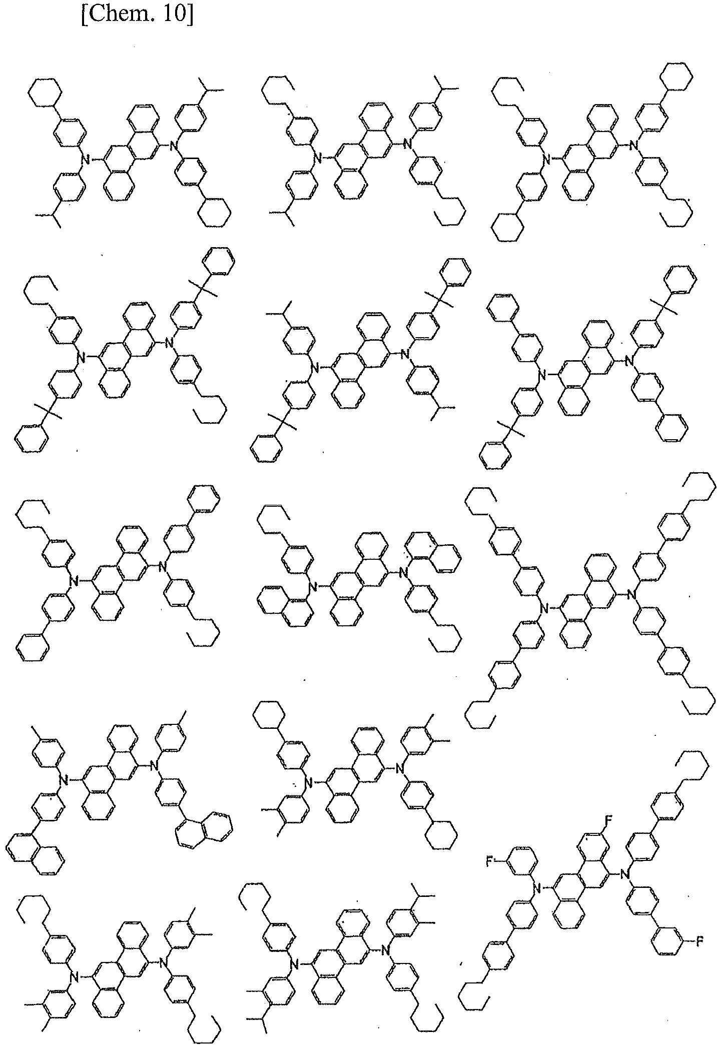

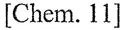

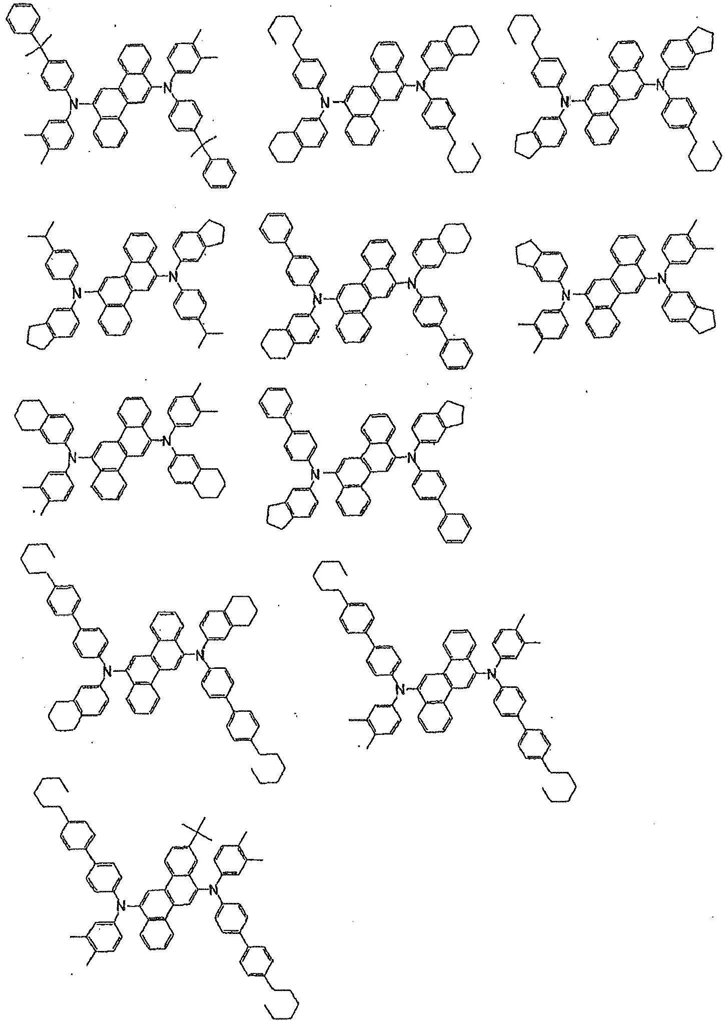

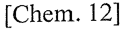

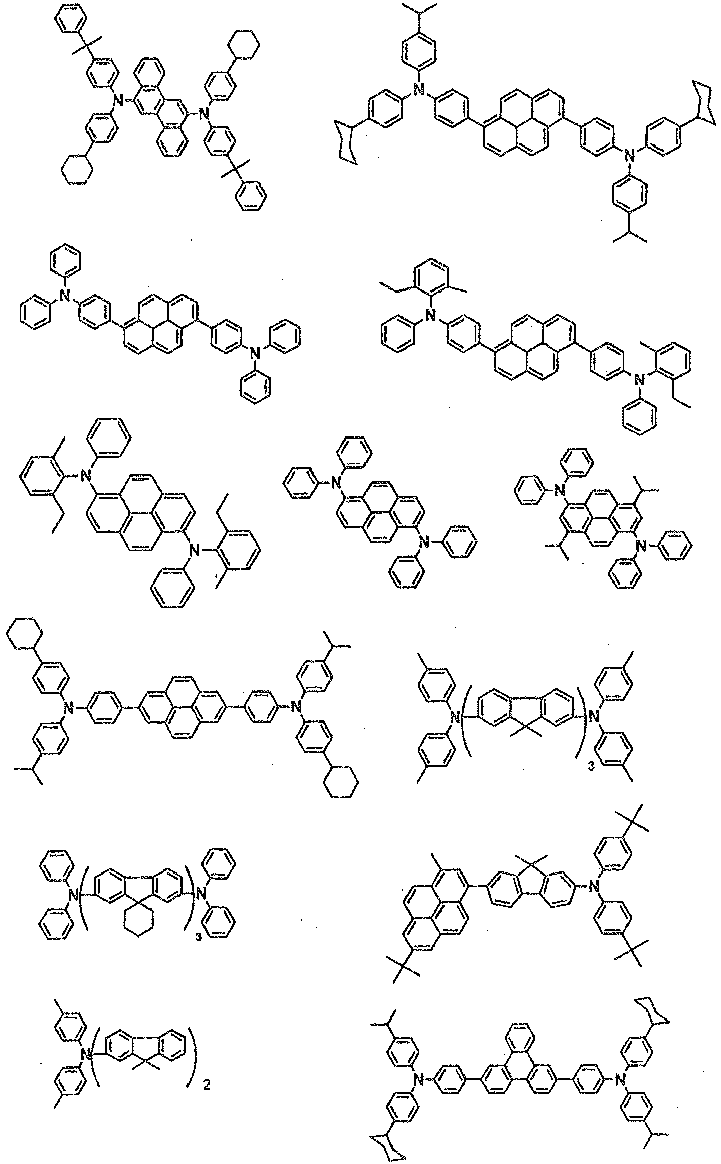

### (Other Components)

The luminescent layer-forming composition for use in the present invention may further contain a coatability improver such as leveling agent, defoaming agent and thickener, an charge transport promoter such as electron-accepting compound and electron-donating compound, a binder resin, and the like. The content of such a component in the luminescent layer-forming composition is usually 50 wt% or less from the standpoint of, for example, not seriously inhibiting the charge transfer of a thin film, not inhibiting luminescence of the luminescent material, or not deteriorating the film quality of a thin film.

### [Film Deposition Method]

In the method for manufacturing an organic electroluminescence element according to the present invention, in the case of forming a coating film by a wet film-forming method, a luminescent layer-forming composition containing the above-described specific low-molecular compound and a solvent is prepared and used.

After wet deposition of the luminescent layer-forming composition, the obtained coating film is dried to remove the solvent, whereby an organic thin film is formed.

The wet film-forming method as used in the present invention indicates a method of performing film deposition by using an ink containing a solvent, such as spin coating method, dip coating method, die coating method, bar coating method, blade coating method, roll coating method, spray coating method, capillary coating method, nozzle printing method, inkjet method, screen printing method, gravure printing method, flexographic printing method and offset printing. In view of ease of patterning, a die coating method, a roll coating method, a spray coating method, an inkjet method, a nozzle printing method, or a flexographic printing method is preferred.

The oxygen concentration in the environment for forming a coating film by a wet film-forming method (hereinafter, sometimes referred to as a "coating step") is usually 18 vol% or more, preferably 19 vol% or more, and is usually 22 vol% or less, preferably 21 vol% or less.
The oxygen concentration in the range above is preferred in terms of the fact that oxygen is appropriately introduced into the luminescent layer and the effects of the present invention are obtained, and also, in the case where the layer below the luminescent layer is an organic layer, is preferred in terms of the fact that oxygen is kept from being excessively introduced into the inside of the organic layer.

The relative humidity in the coating step is not limited as long as the effects of the present invention are not seriously impaired, but the relative humidity is usually 0.01 ppm or more and is usually 80% or less.

Also, the cleanliness in the coating step is, in terms of FED standards, usually Class 100,000 or less, preferably Class 10,000 or less, more preferably Class 1,000 or less, and is usually Class 100 or more, preferably Class 10 or more.
The cleanliness in the range above is preferred because a coating film with little defects is obtained and in turn, an organic electroluminescence element having high driving stability can be manufactured.
Incidentally, the cleanliness can be measured, for example, by using Particle Counter KR-12A (manufactured by RION Co., Ltd., but the present invention is not limited thereto as long as the same measurement can be performed.

The pressure in the coating step is usually 110,000 Pa or less, preferably 105,000 Pa or less, and is usually 90,000 Pa or more, preferably 950,000 Pa or more.
The pressure in the range above is a general atmospheric pressure, and this is advantageous in that equipment, time and the like for creating a vacuum environment are not required and coating of a larger area can be facilitated.
The temperature in the coating step is preferably 10°C or more and preferably 50°C or less so as to prevent the film from a defect due to formation of a crystal in the composition.

After forming the coating film by a wet film-forming method, a step of further heating the coating film (hereinafter, sometimes referred to as a "heating step") is preferably performed.
As the environment of the heating step, an inert gas atmosphere or vacuum is usually employed. The heating step is preferably performed in an inert gas atmosphere in view of production cost and the like and on the other hand, is also preferably performed in vacuum because the process is simple and easy.

Examples of the inert gas include rare gases such as nitrogen gas, helium gas, neon gas, argon gas, krypton gas and xenon gas, and non-combustible gasses such as Freon gas, with a nitrogen gas being preferred.
One of these inert gases may be used, or two or more thereof may be used in arbitrary combination at an arbitrary ratio.

Examples of the heating means used in the heating step include a clean oven, a hot plate, an infrared ray, a halogen heater, and microwave irradiation. Among these, in order to evenly apply heat throughout the film, a clean oven and a hot plate are preferred.
As long as the effects of the present invention are not seriously impaired, the heating temperature in the heating step may be a temperature not lower than the glass transition temperature of the specific lower compound or dopant used in the luminescent layer-forming composition or may be a temperature not more than the glass transition temperature, but usually, the heating is preferably performed at a temperature not more than the melting points of the specific low-molecular compound and the dopant.

In the heating step, the heating time is not limited but is preferably 10 seconds or more and is usually 180 minutes or less. If the heating time is too long, the luminous efficiency is liable to decrease, whereas if the heating time is too short, an inhomogeneous luminescent layer tends to be formed. Heating may be performed in two or more portions.

The film thickness of the organic thin film is arbitrary as long as the effects of the present invention are not seriously impaired, but in the case where the organic thin film is a luminescent layer, the film thickness is usually 3 nm or more, preferably 5 nm or more, and is usually 200 nm or less, preferably 100 nm or less. If the film thickness of the organic thin film is too thin, a defect may be generated in the film, whereas if the film thickness is too thick, the driving voltage may rise.

### [Reason for Producing Effects of the Present Invention]

The reason for producing the effect of the present invention is presumed as follows.
The film density is lower in the film formed by a wet film-forming method than in the film formed by a vacuum deposition method. That is, the number of voids is larger in the film formed by a wet film-forming method than in the film formed by a vapor deposition method, and the void works out to a site for trapping a charge.
Also, in the case of a wet film-forming method, oxygen is adsorbed to the site for trapping a charge, whereby charge movement is liable to be blocked. More specifically, adsorption of oxygen is presumed to cause trapping of particularly an electron.

On the other hand, in the present invention, in view of color and the heat resistance and oxidation-reduction resistance of the compound, the above-described specific compound is used as a charge transport material in the luminescent layer-forming composition, but since the specific low-molecular compound has an anthracene ring in the structure, the luminescent layer containing the compound is considered to allow high electron mobility. As a result, recombination of an electron and a hole occurs nearer the anode, and this is considered to accelerate deterioration in the vicinity of interface between the luminescent layer and an organic layer therebelow (for example, the later-described hole transport layer or hole injection layer) due to driving and probably bring about an adverse effect on the drive life of the organic electroluminescence element.

However, in the present invention, the oxygen concentration at the coating is set to be from 18 to 22 vol%, allowing oxygen to be introduced into the coating film, and an electron is trapped by utilizing an equilibrium reaction of O₂+4e⁻ ⇔ 2O²⁻. As a result, the electron mobility in the luminescent layer is reduced, and it is considered that the luminescence/recombination is shifted nearer the cathode and in turn, the drive life of the organic electroluminescence element is enhanced.

In this connection, furthermore, when a low-boiling-point solvent is used for the luminescent layer-forming composition, the density of the film formed by a wet film-forming method is reduced, and this is considered to more readily allow for adsorption of oxygen in the film than in the case of using a high-boiling-point solvent. This is presumed to occur because of the following reason. The low-boiling-point solvent has a high drying speed and therefore, the solute can hardly intrude into a space formed by removal of the solvent, leading to a low film density. On the other hand, the high-boiling-point solvent has a low drying speed and therefore, the solute readily intrudes into a space formed by removal of the solvent, as a result, the film density becomes high.

After forming the coating film, an organic thin film is formed by heating the coating film, and when the heating is performed in an inert gas atmosphere, a chemical reaction such as oxidation or decomposition by the oxygen in the periphery of the coating film hardly occurs. Therefore, the manufacturing method of the present invention preferably comprises a step of further heating the coating film in an inert gas.
Thanks to these, the organic electroluminescence element formed by the manufacturing method of the present invention is presumed to have a long drive life.

### <Configuration of Organic Electroluminescence Element>

The layer configuration of the organic electroluminescent element of the present invention, the general formation method therefor, and the like are described below by referring to Fig. 1.
Fig. 1 is a schematic cross-sectional view showing a structural example of the organic electroluminescent element according to the present invention. In Fig. 1, 1 denotes a substrate, 2 denotes an anode, 3 denotes a hole injection layer, 4 denotes a hole transport layer, 5 denotes a luminescent layer, 6 denotes a hole blocking layer, 7 denotes an electron transport layer, 8 denotes an electron injection layer, and 9 denotes a cathode.

### {Substrate}

The substrate works out to the support of the organic electroluminescent element, and a quartz or glass plate, a metal plate or foil, a plastic film or sheet, and the like are used therefor. In particular, a glass plate and a transparent plate of a synthetic resin such as polyester, polymethacrylate, polycarbonate and polysulfone are preferred. In the case of using a synthetic resin substrate, gas barrier property must be kept in mind. If the gas barrier property of the substrate is too low, the organic electroluminescent element may be deteriorated due to outside air passed through the substrate, and this is not preferred. Therefore, a method of providing a dense silicon oxide film or the like on at least one surface of the synthetic resin substrate to ensure the gas barrier property is also one of preferred methods.

### {Anode}

The anode fulfills a role of injecting a hole into a layer on the luminescent layer side.
The anode is usually composed of a metal such as aluminum, gold, silver, nickel, palladium and platinum, a metal oxide such as indium and/or tin oxide, a metal halide such as copper iodide, carbon black, or an electrically conductive polymer such as poly(3-methylthiophene), polypyrrole and polyaniline.

Formation of the anode is usually performed by a sputtering method or a vacuum deposition method. In the case of forming the anode by using, for example, a fine metal particle such as silver, a fine particle of copper iodide or the like, carbon black, a fine electrically conductive metal oxide particle or a fine electrically conductive polymer powder, the anode can be also formed by dispersing the fine particle in an appropriate binder resin solution and coating the dispersion on the substrate. Furthermore, in the case of an electrically conductive polymer, the anode can be also formed by forming a thin film directly on the substrate through electrolytic polymerization or coating the electrically conductive polymer on the substrate (see, Appl. Phys. Lett., Vol. 60, page 2711, 1992).

The anode usually has a single-layer structure but, if desired, can be formed to have a multilayer structure composed of a plurality of materials.

The thickness of the anode varies depending on the required transparency. In the case where transparency is required, it is preferred to set the transmittance for visible light to usually 60% or more, preferably 80% or more. In this case, the thickness of the anode is usually 5 nm or more, preferably 10 nm or more, and is usually 1,000 nm or less, preferably on the order of 500 nm or less. In the case where the anode can be opaque, the thickness of the anode is arbitrary, and the anode may be the same as the substrate. Furthermore, a different electrically conductive material may be also stacked on the anode.

For the purpose of removing impurities attached to the anode and adjusting the ionization potential to improve the hole injection performance, the anode surface is preferably subjected to an ultraviolet (UV)/ozone treatment or an oxygen plasma or argon plasma treatment.

### {Hole Injection Layer}

The hole injection layer is a layer for transporting a hole to the luminescent layer from the anode and is usually formed on the anode.
The method for forming the hole injection layer according to the present invention may be either a vacuum deposition method or a wet film-forming method and is not particularly limited, but from the standpoint of reducing the dark spot, the hole injection layer is preferably formed by a wet film-forming method.

The film thickness of the hole injection layer is usually 5 nm or more, preferably 10 nm or more, and is usually 1,000 nm or less, preferably 500 nm or less.

### [Formation of Hole Injection Layer by Wet film-forming method]

In the case of forming the hole injection layer by a wet film-forming method, usually, the materials constituting the hole injection layer are mixed with an appropriate solvent (a hole injection layer solvent) to prepare a composition for film deposition (a hole injection layer-forming composition), and the hole injection layer-forming composition is coated on a layer serving as the underlying layer (usually anode) of the hole injection layer by an appropriate method to deposit a film and then dried, whereby the hole injection layer is formed.

### (Hole Transporting Compound)

The hole injection layer-forming composition usually contains, as the constituent material of the hole injection layer, a hole transporting compound and a solvent.
The hole transporting compound may be a high-molecular compound such as polymer or a low-molecular compound such as monomer, as long as it is a compound having a hole transporting performance usually used for the hole injection layer of an organic electroluminescent element, but a high-molecular compound is preferred.

In view of charge injection barrier from the anode to the hole injection layer, the hole transporting compound is preferably a compound having an ionization potential of 4.5 to 6.0 eV. Examples of the hole transporting compound include an aromatic amine derivative, a phthalocyanine derivative, a porphyrin derivative, an oligothiophene derivative, a polythiophene derivative, a benzylphenyl derivative, a compound having tertiary amines connected through a fluorene group, a hydrazone derivative, a silazane derivative, a silanamine derivative, a phosphamine derivative, a quinacridone derivative, a polyaniline derivative, a polypyrrole derivative, a polyphenylenevinylene derivative, a polythienylenevinylene derivative, a polyquinoline derivative, a polyquinoxaline derivative, and carbon.

Incidentally, the derivative as used in the present invention encompasses, for example, in the case of an aromatic amine derivative, the aromatic amine itself and a compound where the main skeleton is an aromatic amine, and the derivative may be a polymer or a monomer.

For the hole transporting compound used as a material of the hole injection layer, any one of these compounds may be contained alone, or two or more thereof may be contained. In the case of containing two or more hole transporting compounds, the combination thereof is arbitrary, but one kind or two or more kinds of aromatic tertiary amine high-molecular compounds and one kind or two or more kinds of other hole transporting compounds are preferably used in combination.

Among those compounds, in view of amorphous property and transmittance for visible light, an aromatic amine compound is preferred, and an aromatic tertiary amine compound is more preferred. The aromatic tertiary amine compound as used herein is a compound having an aromatic tertiary amine structure and encompasses also a compound having a group derived from an aromatic tertiary amine.

The aromatic tertiary amine compound is not particularly limited in its kind, but in view of uniform luminescence by the surface smoothing effect, a high-molecular compound having a weight average molecular weight of 1,000 to 1,000,000 (a compound of a polymerization type where repeating units are connected) is more preferred. Preferred examples of the aromatic tertiary amine high-molecular compound include a high-molecular compound having a repeating unit represented by the following formula (I):

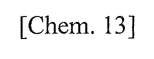

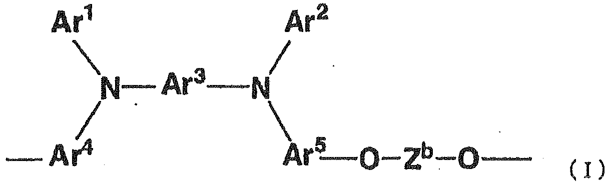

(wherein each of Ar¹ and Ar² independently represents an aromatic hydrocarbon group which may have a substituent, or an aromatic heterocyclic group which may have a substituent, each of Ar³ to Ar⁵ independently represents an aromatic hydrocarbon group which may have a substituent, or an aromatic heterocyclic group which may have a substituent, Z^{b} represents a linking group selected from the following linking groups, and out of Ar¹ to Ar⁵, two groups bonded to the same N atom may combine with each other to form a ring:

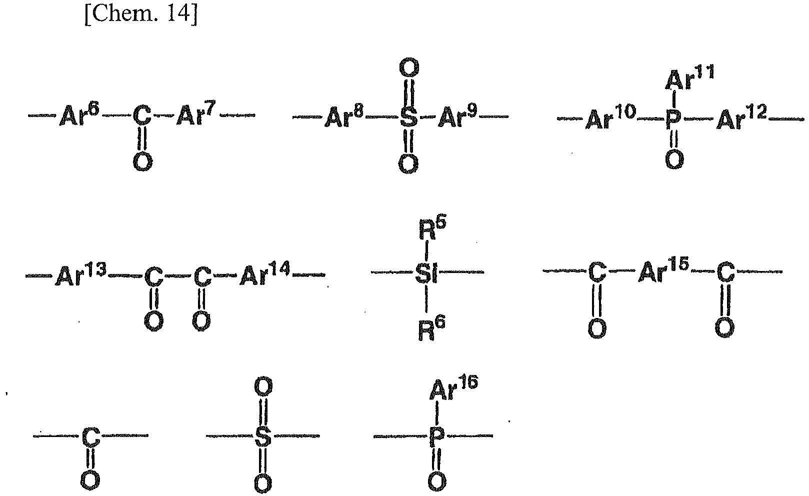

(wherein each of Ar⁶ to Ar¹⁶ independently represent an aromatic hydrocarbon group which may have a substituent, or an aromatic heterocyclic ring which may have a substituent, and each of R⁵ and R⁶ independently represents a hydrogen atom or an arbitrary substituent).

As for the aromatic hydrocarbon group and aromatic heterocyclic group of Ar¹ to Ar¹⁶, in view of solubility, heat resistance and hole injection/transport performance of the high-molecular compound, a group derived from a benzene ring, a naphthalene ring, a phenanthrene ring, a thiophene ring or a pyridine ring is preferred, and a group derived from a benzene ring or a naphthalene ring is more preferred.

The aromatic hydrocarbon group and aromatic heterocyclic group of Ar¹ to Ar¹⁶ may further have a substituent. The molecular weight of the substituent is usually 400 or less, preferably about 250 or less. Preferred examples of the substituent include an alkyl group, an alkenyl group, an alkoxy group, an aromatic hydrocarbon group, and an aromatic heterocyclic group.

In the case where each of R⁵ and R⁶ is an arbitrary substituent, examples of the substituent include an alkyl group, an alkenyl group, an alkoxy group, a silyl group, a siloxy group, an aromatic hydrocarbon group, and an aromatic heterocyclic group.

Specific examples of the aromatic tertiary amine high-molecular compound having a repeating unit represented by formula (I) include the compounds described in International Publication No. 2005/089024.
As the hole transporting compound, an electrically conductive polymer (PEDOT/PSS) obtained by polymerizing 3,4-ethylenedioxythiophene as a polythiophene derivative in a high-molecular-weight polystyrenesulfonic acid is also preferred. Furthermore, the terminal of this polymer may be capped with a methacrylate or the like.

Incidentally, the hole transporting compound may be a crosslinkable polymer described later in [Hole Transport Layer]. The same applied to the film deposition method when using the crosslinkable polymer.

The concentration of the hole transporting compound in the hole injection layer-forming composition is arbitrary as long as the effects of the present invention are not seriously impaired. The concentration is, in view of uniformity of the film thickness, usually 0.01 wt% or more, preferably 0.1 wt% or more, more preferably 0.5 wt% or more, and is usually 70 wt% or less, preferably 60 wt% or less, more preferably 50 wt% or less. If the concentration is too large, film thickness unevenness may occur, whereas if the concentration is too small, a defect may be generated in the hole injection layer deposited.

### (Electron-Accepting Compound)

The hole injection layer-forming composition preferably contains an electron-accepting compound as a constituent material of the hole injection layer.
The electron-accepting compound is preferably a compound having oxidizing power and having an ability to accept one electron from the above-described hole transporting compound. Specifically, a compound having an electron affinity of 4 eV or more is preferred, and a compound having an electron affinity of 5 eV or more is more preferred.
Examples of such an electron-accepting compound include one kind or two or more kinds of compounds selected from the group consisting of a triarylboron compound, a metal halide, a Lewis acid, an organic acid, an onium salt, a salt of arylamine with metal halide, and a salt of arylamine with Lewis acid.

Specific examples thereof is include an organic group-substituted onium salt such as 4-isopropyl-4'-methyldiphenyliodonium tetrakis(pentafluorophenyl)borate and triphenylsulfonium tetrafluoroborate (International Publication No. WO2005/089024); a high-valence inorganic compound such as iron(III) chloride (JP-A-11-251067) and ammonium peroxodisulfate; a cyano compound such as tetracyanoethylene; an aromatic boron compound such as tris(pentafluorophenyl)borane (JP-A-2003-31365); a fullerene derivative; iodine; and a sulfonate ion such as polystyrenesulfonate ion, alkylbenzenesulfonate ion and camphorsulfonate ion.
Such an electron-accepting compound can enhance the electric conductivity of the hole injection layer by oxidizing the hole transporting compound.

The content of the electron-accepting compound in the hole injection layer or the hole injection layer-forming composition is, based on the hole transporting compound, usually 0.1 mol% or more, preferably 1 mol% or more, and is usually 100 mol% or less, preferably 40 mol% or less.

### (Other Constituent Materials)

As the material of the hole injection layer, in addition to the above-described hole transporting compound and electron-accepting compound, other components may be further incorporated as long as the effects of the present invention are not seriously impaired. Examples of other components include various luminescent materials, electron transporting compounds, binder resins and coatability improvers. Incidentally, as for other components, only one component may be used, or two or more components may be used in arbitrary combination at an arbitrary ratio.

### (Solvent)

Out of the solvents in the hole injection layer-forming composition used for a wet film-forming method, at least one solvent is preferably a compound capable of dissolving the above-described constituent materials of the hole injection layer. The boiling point of this solvent is usually 110°C or more, preferably 140°C or more, more preferably 200°C or more, and is usually 400°C or less, preferably 300°C or less. If the boiling point of the solvent is too low, the drying speed is excessively high and the film quality may be impaired, whereas if the boiling point of the solvent is too high, the temperature in the drying step must be raised and this may adversely affect other layers or the substrate.

Examples of the solvent include an ether-based solvent, an ester-based solvent, an aromatic hydrocarbon-based solvent, and an amide-based solvent.

Examples of the ether-based solvent include an aliphatic ether such as ethylene glycol dimethyl ether, ethylene glycol diethyl ether and propylene glycol-1-monomethyl ether acetate (PGMEA); and an aromatic ether such as 1,2-dimethoxybenzene, 1,3-dimethoxybenzene, anisole, phenetole, 2-methoxytoluene, 3-methoxytoluene, 4-methoxytoluene, 2,3-dimethylanisole and 2,4-dimethylanisole.

Examples of the ester-based solvent include an aromatic ester such as phenyl acetate, phenyl propionate, methyl benzoate, ethyl benzoate, propyl benzoate and n-butyl benzoate.

Examples of the aromatic hydrocarbon-based solvent include toluene, xylene, cyclohexylbenzene, 3-isopropylbiphenyl, 1,2,3,4-tetramethylbenzene, 1,4-diisopropylbenzene, cyclohexylbenzene and methylnaphthalene.

Examples of the amide-based solvent include N,N-dimethylformamide and N,N-dimethylacetamide.

In addition, dimethylsulfoxide and the like may be also used.
Only one of these solvents may be used, or two or more thereof may be used in arbitrary combination at an arbitrary ratio.

### (Film Deposition Method)

After the preparation of the hole injection layer-forming composition, the composition is coated/deposited by a wet film-forming method on a layer serving as the underlying layer (usually anode) of the hole injection layer and dried, whereby the hole injection layer is formed.
The temperature in the coating step is preferably 10°C or more and preferably 50°C or less so as to prevent the film from a defect due to formation of a crystal in the composition.
The relative humidity in the coating step is not limited as long as the effects of the present invention are not seriously impaired, but the relative humidity is usually 0.01 ppm or more and usually 80% or less.

After the coating, the film of the hole injection layer-forming composition is usually dried by heating or the like. Examples of the heating means used in the heating step include a clean oven, a hot plate, an infrared ray, a halogen heater, and microwave irradiation. Among these, in order to evenly apply heat throughout the film, a clean oven and a hot plate are preferred.

As for the heating temperature in the heating step, as long as the effects of the present invention are not seriously impaired, the heating is preferably performed at a temperature not lower than the boiling point of the solvent used in the hole injection layer-forming composition. In the case where the solvent used in the hole injection layer is a mixed solvent containing two or more kinds of solvents, the heating is preferably performed at a temperature not lower than the boiling point of at least one kind of the solvent. Considering a rise in the boiling point of the solvent, heating in the heating step is preferably performed at 120°C or more and preferably at 410°C or less.

In the heating step, the heating time is not limited as long as the heating temperature is not lower than the boiling point of the solvent in the hole injection layer-forming composition and full insolubilization of the coating film does not occur, but the heating time is preferably 10 seconds or more and is usually 180 minutes or less. If the heating time is too long, components in other layers may diffuse, whereas if it is too short, the hole injection layer is likely to become inhomogeneous. Heating may be performed in two or more portions.

### [Formation of Hole Injection Layer by Vacuum Deposition Method]

In the case of forming the hole injection layer by vacuum deposition, one material or two or more materials out of the constituent materials (for example, the above-described hole transporting compound and electron-accepting compound) of the hole injection layer are put in a crucible (in the case of using two or more materials, in respective crucibles) placed in a vacuum vessel, and the inside of the vacuum vessel is evacuated to about 10⁻⁴ Pa by an appropriate vacuum pump. The crucible is then heated (in the case of using two or more materials, respective crucibles are heated) to cause evaporation by controlling the evaporated amount (in the case of using two or more materials, by independently controlling the evaporated amount of each material), whereby a hole injection layer is formed on the anode of the substrate placed to face the crucible. Incidentally, in the case of using two or more materials, the hole injection layer may be also formed by putting a mixture of these materials in a crucible and heating it to cause evaporation.

The degree of vacuum at the vapor deposition is not limited as long as the effects of the present invention are not seriously impaired, but the degree of vacuum is usually 0.1×10⁻⁶ Torr (0.13×10⁻⁴ Pa) or more and usually 9.0×10⁻⁶ Torr (12.0×10⁻⁴ Pa) or less. The vapor deposition rate is not limited as long as the effects of the present invention are not seriously impaired, but the vapor deposition rate is usually 0.1 Å/sec or more and usually 5.0 A/sec or less. The film deposition temperature at the vapor deposition is not limited as long as the effects of the present invention are not seriously impaired, but the vapor deposition is performed preferably at 10°C or more and preferably at 50°C or less.

### {Hole Transport Layer}

The method for forming the hole transport layer according to the present invention may be either a vacuum deposition method or a wet film-forming method and is not particularly limited, but from the standpoint of reducing the dark spot, the hole transport layer is preferably formed by a wet film-forming method.
The hole transport layer can be formed on the hole injection layer in the case of having a hole injection layer and can be formed on the anode in the case of not having a hole injection layer. Also, the organic electroluminescence element of the present invention may have a configuration where the hole transport layer is omitted.

The material for forming the hole transport layer is preferably a material having a high hole transporting performance and being capable of efficiently transporting the injected hole. Accordingly, a material having a small ionization potential, high transparency to visible light, large hole mobility and excellent stability and hardly allowing impurities working out to a trap to be generated during production or use is preferred. Also, the hole transport layer contacts with the luminescent layer in many cases and therefore, the material is preferably kept from quenching the light emitted from the luminescent layer or reducing the efficiency by forming an exciplex with the luminescent layer.

Such a material for the hole transport layer may be sufficient if it is a material conventionally used as a constituent material of the hole transport layer, and examples thereof include those described above as examples of the hole transporting compound used in the hole injection layer. Other examples include an arylamine derivative, a fluorene derivative, a spiro derivative, a carbazole derivative, a pyridine derivative, a pyrazine derivative, a pyrimidine derivative, a triazine derivative, a quinoline derivative, a phenanthroline derivative, a phthalocyanine derivative, a porphyrin derivative, a silole derivative, an oligothiophene derivative, a condensed polycyclic aromatic derivative, and a metal complex.

Furthermore, examples of the material include a polyvinylcarbazole derivative, a polyarylamine derivative, a polyvinyltriphenylamine derivative, a polyfluorene derivative, a polyarylene derivative, a tetraphenylbenzidine-containing polyarylene ether sulfone derivative, a polyarylenevinylene derivative, a polysiloxane derivative, a polythiophene derivative, and a poly(p-phenylenevinylene) derivative. These may be any of an alternating copolymer, a random copolymer, a block copolymer and a graft copolymer, and may be also a polymer having a branch in the main chain and having three or more terminals, a so-called dendrimer.

Among these, a polyarylamine derivative and a polyarylene derivative are preferred.
The polyarylamine derivative is preferably a polymer containing a repeating unit represented by the following formula (II). Above all, a polymer composed of repeating units represented by the following formula (II) is preferred, and in this case, Ar^{a} or Ar^{b} may differ among respective repeating units.

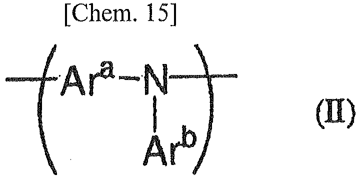

(wherein each of Ar^{a} and Ar^{b} independently represents an aromatic ring group or an aromatic heterocyclic group, which may have a substituent).

Examples of the aromatic hydrocarbon group which may have a substituent include a group derived from a 6-membered monocyclic ring or a 2- to 5-fused ring, such as benzene ring, naphthalene ring, anthracene ring, phenanthrene ring, perylene ring, tetracene ring, pyrene ring, benzopyrene ring, chrysene ring, triphenylene ring, acenaphthene ring, fluoranthene ring and fluorene ring, and a group formed by connecting two or more of these rings by direct bonding.

Examples of the aromatic heterocyclic group which may have a substituent include a group derived from a 5- or 6-membered monocyclic ring or a 2- to 4-fused ring, such as furan ring, benzofuran ring, thiophene ring, benzothiophene ring, pyrrole ring, pyrazole ring, imidazole ring, oxadiazole ring, indole ring, carbazole ring, pyrroloimidazole ring, pyrrolopyrazole ring, pyrrolopyrrole ring, thienopyrrole ring, thienothiophene ring, furopyrrole ring, furofuran ring, thienofuran ring, benzisoxazole ring, benzisothiazole ring, benzimidazole ring, pyridine ring, pyrazine ring, pyridazine ring, pyrimidine ring, triazine ring, quinoline ring, isoquinoline ring, cinnoline ring, quinoxaline ring, phenanthridine ring, benzimidazole ring, perimidine ring, quinazoline ring, quinazolinone ring and azulene ring, and a group formed by connecting two or more of these rings by direct bonding.

In view of solubility for an organic solvent and heat resistance, each of Ar^{a} and Ar^{b} is independently, preferably a group derived from a ring selected from the group consisting of a benzene ring, a naphthalene ring, an anthracene ring, a phenanthrene ring, a triphenylene ring, a pyrene ring, a thiophene ring, a pyridine ring and a fluorene ring, or a group formed by connecting two or more benzene rings (for example, a biphenyl group or a terphenylene group).
Above all, a group derived from a benzene ring (phenyl group), a group formed by connecting two benzene rings (biphenyl group), and a group derived from a fluorene ring (fluorenyl group) are preferred.

Examples of the substituent which may be substituted on the aromatic hydrocarbon group and aromatic heterocyclic group of Ar^{a} and Ar^{b} include an alkyl group, an alkenyl group, an alkynyl group, an alkoxy group, an aryloxy group, an alkoxycarbonyl group, a dialkylamino group, a diarylamino group, an acyl group, a halogen atom, a haloalkyl group, an alkylthio group, an arylthio group, a silyl group, a siloxy group, a cyano group, an aromatic hydrocarbon ring group, and an aromatic heterocyclic group.

The polyarylene derivative includes a polymer having, in its repeating unit, an arylene group such as aromatic hydrocarbon group and aromatic heterocyclic group, each of which may have a substituent, described as examples for Ar^{a} or Ar^{b} in formula (II).

The polyarylene derivative is preferably a polymer having a repeating unit composed of the following formula (III-1) and/or the following formula (III-2):

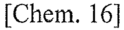

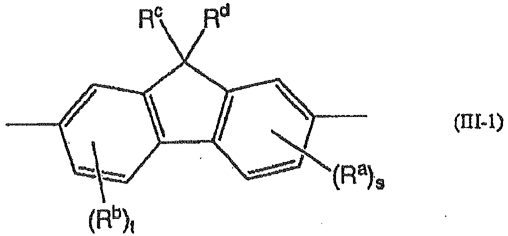

(wherein each of R^{a}, R^{b}, R^{c} and R^{d} independently represents an alkyl group, an alkoxy group, a phenylalkyl group, a phenylalkoxy group, a phenyl group, a phenoxy group, an alkylphenyl group, an alkoxyphenyl group, an alkylcarbonyl group, an alkoxycarbonyl group or a carboxy group, each of t and s independently represents an integer of 0 to 3, provided that when t or s is 2 or more, the plurality of R^{a}s or R^{b}s contained per molecule may be the same or different and adjacent R^{a}s or R^{b}s may form a ring);

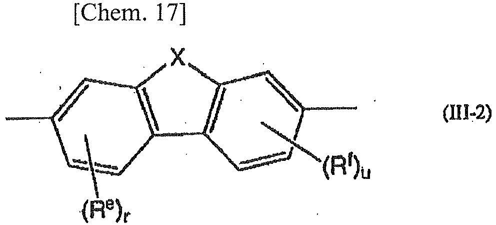

(wherein each of R^{e} and R^{f} independently has the same meaning as R^{a}, R^{b}, R^{c} or R^{d} in formula (III-1), each of r and u independently represents an integer of 0 to 3, provided that when r or u is 2 or more, the plurality of R^{e}s or R^{f}s contained per molecule may be the same or different and adjacent R^{e}s or R^{f}s may form a ring, and X represents an atom or atomic group constituting a 5-membered ring or a 6-membered ring).

Specific examples of X include -O-, -BR-, -NR-, -SiR₂-, -PR-, -SR-, -CR₂-, and a group formed by combining these. R represents a hydrogen atom or an arbitrary organic group. The organic group as used in the present invention is a group containing at least one carbon atom.

The polyarylene derivative preferably further contains a repeating unit represented by the following formula (III-3), in addition to the repeating unit composed of formula (III-1) and/or formula (III-2):

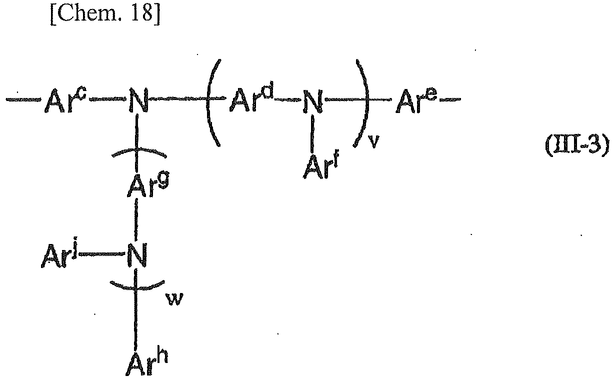

(wherein each of Ar^{c} to Ar^{j} independently represents an aromatic hydrocarbon group or an aromatic heterocyclic group, which may have a substituent, and each of v and w independently represents 0 or 1).

Specific examples of Ar^{c} to Ar^{j} are the same as those of Ar^{a} and Ar^{b} in formula (II).

Specific examples of formulae (III-1) to (III-3), specific examples of the polyarylene derivative, and the like include those described in JP-A-2008-98619.

In the case of forming the hole transport layer by a wet film-forming method, similarly to formation of the hole injection layer, a hole transport layer-forming composition is prepared, wet deposited and then dried by heating.
The hole transport layer-forming composition contains a solvent in addition to the above-described hole transporting compound. The solvent used is the same as the solvent used for the hole injection layer-forming composition. Furthermore, the film deposition conditions, heating/drying conditions and the like are also the same as those in the formation of the hole injection layer.

Also in the case of forming the hole transport layer by a vacuum deposition method, the film deposition conditions and the like are the same as those in the formation of the hole injection layer.
The hole transport layer may contain various luminescent materials, electron transporting compounds, binder resins, coatability improvers and the like, in addition to the hole transporting compound.

The hole transport layer may be a layer formed by crosslinking a crosslinkable compound. The crosslinkable compound is a compound having a crosslinkable group and forms a network high-molecular compound by undergoing crosslinking.
Examples of the crosslinkable group include a group derived from a cyclic ether such as oxetane and epoxy; a group derived from an unsaturated double bond, such as vinyl group, trifluorovinyl group, styryl group, acryl group, methacryloyl and cinnamoyl; and a benzocyclobutane-derived group.

The crosslinkable compound may be any of a monomer, an oligomer and a polymer. Only one crosslinkable compound may be used, or two or more crosslinkable compounds may be used in arbitrary combination at an arbitrary ratio.
As the crosslinkable compound, a hole transporting compound having a crosslinkable group is preferably used. The hole transporting compound includes those exemplified above, and the crosslinkable compound includes compounds where a crosslinkable group is bonded to the main or side chain of those hole transporting compounds. In particular, the crosslinkable group is preferably bonded to the main chain through a linking group such as alkylene group. Above all, the hole transporting compound is preferably a polymer containing a repeating unit having a crosslinkable group and is preferably a polymer containing a repeating unit where a crosslinkable group is bonded to formula (II) or formulae (III-1) to (III-3) directly or through a linking group.

As the crosslinkable compound, a hole transporting compound having a crosslinkable group is preferably used. Examples of the hole transporting compound include a nitrogen-containing aromatic compound derivative such as pyridine derivative, pyrazine derivative, pyrimidine derivative, triazine derivative, quinoline derivative, phenanthroline derivative, carbazole derivative, phthalocyanine derivative and porphyrin derivative; a triphenylamine derivative; a silole derivative; an oligothiophene derivative, a condensed polycyclic aromatic derivative, and a metal complex. Among these, for example, a nitrogen-containing aromatic derivative such as pyridine derivative, pyrazine derivative, pyrimidine derivative, triazine derivative, quinoline derivative, phenanthroline derivative and carbazole derivative; a triphenylamine derivative, a silole derivative, a condensed polycyclic aromatic derivative and a metal complex are preferred, and a triphenylamine derivative is more preferred.

For forming the hole transport layer by crosslinking a crosslinkable compound, usually, a hole transport layer-forming composition obtained by dissolving or dispersing a crosslinkable compound in a solvent is prepared, and the composition is deposited as a film by wet deposition and then crosslinked.

The hole transport layer-forming composition may contain an additive for accelerating the crosslinking reaction, other than the crosslinkable compound. Examples of the additive for accelerating the crosslinking reaction include a polymerization initiator and a polymerization accelerator, such as alkylphenone compound, acylphosphine oxide compound, metallocene compound, oxime ester compound, azo compound and onium salt; and a photosensitizer such as condensed polycyclic hydrocarbon, porphyrin compound and diaryl ketone compound.

Furthermore, the composition may also contain a coatability improver such as leveling agent and defoaming agent; an electron-accepting compound; a binder resin; and the like.

The hole transport layer-forming composition contains the crosslinkable compound in an amount of usually 0.01 wt% or more, preferably 0.05 wt% or more, more preferably 0.1 wt% or more, and usually 50 wt% or less, preferably 20 wt% or less, more preferably 10 wt% or less.

The hole transport layer-forming composition containing a crosslinkable compound in such a concentration is deposited on the underlying layer (usually, the hole injection layer) and then, the crosslinkable compound is crosslinked under heating and/or irradiation with active energy such as light to form a network high-molecular compound.

The conditions such as temperature and humidity at the film deposition are the same as those at the wet deposition of the hole injection layer.
The method for heating after film deposition is not particularly limited. The heating temperature condition is usually 120°C or more and is preferably 400°C or less.
The heating time is usually 1 minute or more and is preferably 24 hours or less. The heating method is not particularly limited, but, for example, a method of placing a laminate having the deposited layer on a hot plate or heating the laminate in an oven is used. For example, conditions such as heating on a hot plate at 120°C or more for 1 minute or more may be employed.

In the case of irradiation with active energy such as light, examples of the method therefor include a method of irradiating the composition directly with an ultraviolet/visible/infrared light source such as ultrahigh pressure mercury lamp, high pressure mercury lamp, halogen lamp and infrared lamp, and a method of irradiating the composition by using a mask aligner having incorporated thereinto the light source above or using a conveyor-type light irradiation apparatus. In the case of irradiation with active energy other than light, examples of the method therefor include a method of irradiating the composition by using an apparatus for delivering a microwave generated by a magnetron, that is, a so-called microwave oven.

As for the irradiation time, a condition necessary to reduce the solubility of the film is preferably set, but irradiation is performed for usually 0.1 seconds or more and preferably 10 hours or less.
Heating and irradiation with active energy such as light may be performed individually or in combination. In the case of combination, the order of practicing these means is not particularly limited.

The film thickness of the thus-formed hole transport layer is usually 5 nm or more, preferably 10 nm or more, and is usually 300 nm or less, preferably 100 nm or less.

### {Luminescent layer}

A luminescent layer is provided on the hole injection layer or when a hole transport layer is provided, on the hole transport layer.
The luminescent layer in the present invention is formed according to the above-described film deposition method by using the above-described luminescent layer-forming composition.

The film thickness of the luminescent layer is arbitrary as long as the effects of the present invention are not seriously impaired, but the thickness is usually 3 nm or more, preferably 5 nm or more, and is usually 200 nm or less, preferably 100 nm or less. If the film thickness of the luminescent layer is too thin, a defect may be generated in the film, whereas if it is too thick, the driving voltage may rise.

### {Hole Blocking Layer}

A hole blocking layer may be provided between the luminescent layer and the later-described electron injection layer. The hole blocking layer is a layer usually stacked on the luminescent layer to come into contact with the interface on the cathode side of the luminescent layer.
The hole blocking layer has a role of blocking a hole migrating from the anode to reach the cathode and a role of efficiently transporting an electron injected from the cathode in the direction toward the luminescent layer.

Physical properties required of the material constituting the hole blocking layer include high electron mobility, low hole mobility, large energy gap (difference between HOMO and LUMO), and high triplet excited level (T1). Examples of the hole blocking layer material satisfying these conditions include a mixed ligand complex such as bis(2-methyl-8-quinolinolato)(phenolato)aluminum and bis(2-methyl-8-quinolinolato)(triphenylsilanolato)aluminum, a metal complex such as bis(2-methyl-8-quinolate)aluminum-µ-oxo-bis-(2-methyl-8-quinolilato)aluminum binuclear metal complex, a styryl compound such as distyrylbiphenyl derivative (JP-A-11-242996), a triazole derivative such as 3-(4-biphenylyl)-4-phenyl-5(4-tert-butylphenyl)-1,2,4-triazole (JP-A-7-41759), and a phenanthroline derivative such as bathocuproine (JP-A-10-79297). Furthermore, a compound having at least one pyridine ring substituted at 2-, 4- and 6-positions described in International Publication No. 2005-022962 is also preferred as the material of the hole blocking layer.

As the material of the hole blocking layer, only one material may be used, or two or more materials may be used in arbitrary combination at an arbitrary ratio.
The method for forming the hole blocking layer is not limited. Accordingly, the layer can be formed by a wet film-forming method, a vapor deposition method or other methods.

The film thickness of the hole blocking layer is arbitrary as long as the effects of the present invention are not seriously impaired, but the film thickness is usually 0.3 nm or more, preferably 0.5 nm or more, and is usually 100 nm or less, preferably 50 nm or less.

### {Electron Transport Layer}

An electron transport layer may be provided between the luminescent layer and the later-described electron injection layer.
The electron transport layer is a layer provided for the purpose of more enhancing the luminous efficiency of the element and is formed of a compound capable of efficiently transporting an electron injected from the cathode in the direction toward the luminescent layer between the electrodes to which an electric field is applied.

As the electron transporting compound used for the electron transport layer, a compound having high electron injection efficiency from the cathode or the electron injection layer and high electron mobility and being capable of efficiently transporting the injected electron is usually used. Examples of the compound satisfying these conditions include a metal complex such as aluminum complex of 8-hydroxyquinoline (JP-A-59-194393), a metal complex of 10-hydroxybenzo[h]quinoline, an oxadiazole derivative, a distyrylbiphenyl derivative, a silole derivative, a 3-hydroxyflavone metal complex, a 5-hydroxyflavone metal complex, a benzoxazole metal complex, a benzothiazole metal complex, trisbenzimidazolylbenzene (U.S. Patent 5,645,948), a quinoxaline compound (JP-A-6-207169), a phenanthroline derivative (JP-A-5-331459), 2-tert-butyl-9,10-N,N'-dicyanoanthraquinonediimine, n-type hydrogenated amorphous silicon carbide, n-type zinc sulfide, and n-type zinc selenide.
As the material of the electron transport layer, only one material may be used, or two or more materials may be used in arbitrary combination at an arbitrary ratio.

The method for forming the electron transport layer is not limited. Accordingly, the layer can be formed by a wet deposition process, a vapor deposition method or other methods.

The film thickness of the electron transport layer is arbitrary as long as the effects of the present invention are not seriously impaired, but the film thickness is usually 1 nm or more, preferably 5 nm or more, and is usually 300 nm or less, preferably 100 nm or less.

### {Electron Injection Layer}

The electron injection layer fulfills a role of efficiently injecting an electron injected from the cathode, into the luminescent layer. In order to efficiently perform the electron injection, the material forming the electron injection layer is preferably a metal having a low work function. Examples thereof include an alkali metal such as sodium and cesium, and an alkaline earth metal such as barium and calcium. The film thickness of the layer is usually 0.1 nm or more and is preferably 5 nm or less.

An organic electron transport compound typified by a nitrogen-containing heterocyclic compound such as bathophenanthroline and a metal complex such as aluminum complex of 8-hydroxyquinoline is preferably doped with an alkali metal such as sodium, potassium, cesium, lithium and rubidium (described, for example, in JP-A-10-270171, JP-A-2002-100478 and JP-A-2002-100482), because both enhanced electron injection/transport performance and excellent film quality can be achieved.
In this case, the film thickness is usually 5 nm or more, preferably 10 nm or more, and is usually 200 nm or less, preferably 100 nm or less.
As the material of the electron injection layer, only one material may be used, or two or more materials may be used in arbitrary combination at an arbitrary ratio.

The method for forming the electron injection layer is not limited. Accordingly, the layer can be formed by a wet deposition process, a vapor deposition method or other methods.

### {Cathode}

The cathode fulfills a role of injecting an electron into a layer (for example, the electron injection layer or the luminescent layer) on the luminescent layer side.

As the material of the cathode, materials used for the above-described anode may be used, but in order to efficiently perform the electron injection, the material is preferably a metal having a low work function and, for example, an appropriate metal such as tin, magnesium, indium, calcium, aluminum and silver, or an alloy thereof is used. Specific examples thereof include an alloy electrode having a low work function, such as magnesium-silver alloy, magnesium-indium alloy and aluminum-lithium alloy.
Incidentally, as the material of the cathode, only one material may be used, or two or more materials may be used in arbitrary combination at an arbitrary ratio.

The film thickness of the cathode is usually the same as that of the anode.

For the purpose of protecting the cathode composed of a metal having a low work function, a metal layer having a high work function and being stable to the atmosphere is preferably further stacked thereon, because the stability of the element is increased. For example, a metal such as aluminum, silver, copper, nickel, chromium, gold and platinum is used to this effect.
As such a material, only one material may be used, or two or more materials may be used in arbitrary combination at an arbitrary ratio.

### {Other Layers}

The organic electroluminescence element according to the present invention may have other configurations without departing from the scope of the invention and, for example, may have an optional layer between the anode and the cathode, in addition to the layers described above as long as the performance thereof is not impaired. Also, an arbitrary layer may be omitted.

### [Electron Blocking Layer]

The layer which the element may have includes, for example, an electron blocking layer.
The electron blocking is provided between the hole injection layer or hole transport layer and the luminescent layer and has a role of blocking an electron migrating from the luminescent layer to reach the hole injection layer, thereby increasing the probability of recombination of a hole and an electron in the luminescent layer and confining the produced exciton in the luminescent layer, and a role of efficiently transporting a hole injected from the hole injection layer in the direction toward the luminescent layer. In particular, when a phosphorescent material or a blue luminescent material is used as the luminescent material, it is effective to provide an electron blocking layer.

The characteristics required of the electron blocking layer include, for example, high hole transporting performance, large energy gap (difference between HOMO and LUMO), and high triplet excited level (T1). Furthermore, in the present invention, when the luminescent layer is produced as the organic layer according to the present invention by a wet film-forming method, the electron blocking layer is also required to have suitability for wet deposition. Examples of the material used for such an electron blocking layer include a copolymer of dioctylfluorene and triphenylamine, typified by F8-TFB (International Publication No. 2004/084260).

As the material of the electron blocking layer, only one material may be used, or two or more materials may be used in arbitrary combination at an arbitrary ratio.
The method for forming the electron blocking layer is not limited. Accordingly, the layer can be formed by a wet film-forming method, a vapor deposition method or other methods.

It is also an effective method for enhancing the element efficiency to insert an ultrathin insulating film (from 0.1 to 5 nm) formed of, for example, lithium fluoride (LiF), magnesium fluoride (MgF₂), lithium oxide (Li₂O) or cesium(II) carbonate (CsCO₃) into the interface between the cathode and the luminescent layer or electron transport layer (see, for example, Applied Physics Letters, Vol. 70, page 152 (1997); JP-A-10-74586; IEEE Transactions on Electron Devices, Vol. 44, page 1245 (1997); and SID 04 Digest, page 154).

In the layer configuration described above, the constituent elements other than the substrate may be also stacked in reverse order. For example, in the layer configuration of Fig. 1, on the substrate, other constituent elements may be provided in order of a cathode, an electron injection layer, an electron transport layer, a hole blocking layer, a luminescent layer, a hole transport layer, a hole injection layer and an anode.

Furthermore, the organic electroluminescence element according to the present invention may also have a configuration where constituent elements other than the substrate are stacked between two substrates with at least one substrate having transparency.

A structure where constituent elements (light-emitting unit) other than the substrate are stacked in a plurality of tiers (a structure where a plurality of light-emitting units are stacked) may be also employed. In this case, when a carrier generation layer (CGL) composed of, for example, vanadium pentoxide (V₂O₅) is provided in place of interface layers (when the anode is ITO and the cathode is Al, these two layers) between respective tiers (between light-emitting units), the barrier between tiers is reduced and this more preferred in view of luminous efficiency and driving voltage.

In addition, the organic electroluminescence element according to the present invention may be configured as a single organic electroluminescence element, may be applied to a configuration where a plurality of organic electroluminescence elements are arranged in an array manner, or may be applied to a configuration where the anode and the cathode are arranged in an X-Y matrix manner.

In each of the above-described layers, components other than those described as the material may be contained as long as the effects of the present invention are not seriously impaired.

### <Display Device>

The display device of the present invention has the above-described organic electroluminescence element of the present invention. The display device of the present invention is not particularly limited in its mode or structure and can be fabricated according to a conventional method by using the organic electroluminescence element of the present invention.
For example, the display device of the present invention can be formed by such a method as described in Seishi Tokito, Chihaya Adachi and Hideyuki Murata, Yuki EL Display (Organic EL Display), Ohm-Sha (August 20, 2004).

### <Lighting Device>

The lighting device of the present invention is a lighting device having the above-described organic electroluminescence element of the present invention. The lighting device of the present invention is not particularly limited in its mode or structure and can be fabricated according to a conventional method by using the organic electroluminescence element of the present invention.

### EXAMPLES

The present invention is described in greater detail below by referring to Examples, but the present invention is not limited to the following Examples as long as the gist of the present invention is observed.

### (Example 1)

An organic electroluminescence element having the structure shown in Fig. 1 was manufactured by the following method.
A glass substrate 1 having deposited thereon an indium/tin oxide (ITO) transparent electrically conductive film to a thickness of 150 nm (sputtering-deposited product, sheet resistance: 15 Ω) was patterned with 2 mm-wide stripes by using normal photolithography technique and hydrochloric acid etching to form an anode 2. The pattern-formed ITO substrate was washed successively by ultrasonic cleaning with acetone, washing with pure water, and ultrasonic cleaning with isopropyl alcohol, then dried by nitrogen blowing, and finally subjected to an ultraviolet-ozone cleaning.

Next, a hole injection layer 3 was formed by a wet film-forming method as follows. An aromatic amino group-containing charge transport material (PB-1 (weight average molecular weight: 52,000, number average molecular weight: 32,500)) having a structural formula shown below and an electron-accepting compound (A-2) having a structural formula shown below were used as the material of the hole injection layer 3, and the composition was spin-coated under the following conditions.

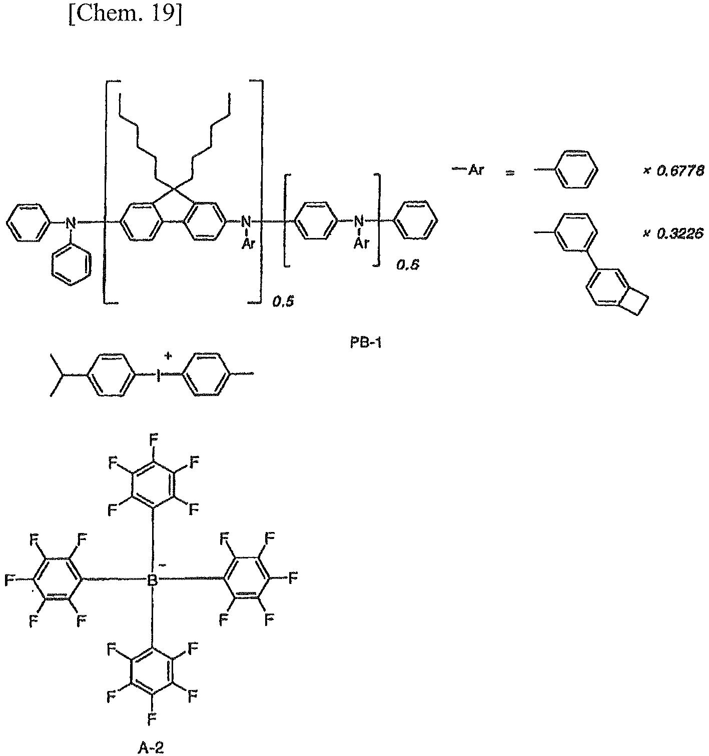

### <Composition for Hole Injection Layer>

### Solvent: ethyl benzoate

| | |
|---|---|
| Coating solution concentration: | PB-1: 2.0 wt% |
| | A-2: 0.4 wt% |

### <Film Deposition Conditions>

Spinner rotation speed: 2,250 rpm
Spinner rotation time: 30 seconds
Spin coating atmosphere: in the atmosphere
Bake conditions: 230°Cx60 minutes (in the atmosphere)
By the spin coating above, a uniform thin film having a thickness of 30 nm was formed.

Subsequently, a hole transport layer was formed by a wet film-forming method as follows. An organic electroluminescence element composition using a charge transport material (PB-2) having a structural formula shown below as the material of the hole transport layer and cyclohexylbenzene as the solvent was prepared, and this organic electroluminescence element composition was spin-coated under the following conditions.

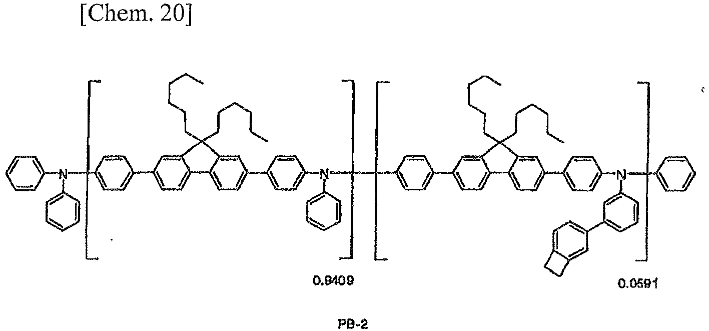

### <Composition for Hole Transport Layer>

| | |
|---|---|
| Solvent: cyclohexylbenzene | |
| Coating solution concentration: | PB-2:1.4 wt% |

### <Film Deposition Conditions>

Spinner rotation speed: 1,500 rpm
Spinner rotation time: 120 seconds
Spin coating atmosphere: in the atmosphere
Bake conditions: 230°Cx60 minutes (in dry nitrogen)
By the spin coating above, a uniform thin film having a thickness of 20 nm was formed.

Next, in forming a luminescent layer, an organic electroluminescence element composition shown below was prepared by using an organic compound (HO-1) shown below as the charge transport material and an organic compound (DO-1) shown below as the luminescent material and spin-coated on the hole transport layer under the following conditions to obtain a luminescent layer having a film thickness of 40 nm.

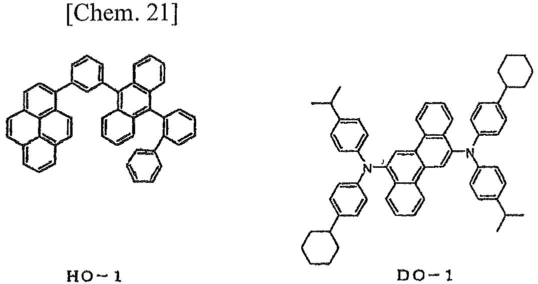

### <Luminescent layer-Forming Composition>

| | |
|---|---|
| Solvent: cyclohexylbenzene | |
| Concentration in composition: | HO-1: 5 wt% |
| | DO-1: 0.5 wt% |

### <Film Deposition Conditions>

Spinner rotation speed: 2,000 rpm
Spinner rotation time: 120 seconds
Spin coating atmosphere: oxygen concentration of 20%, 25°C, 1.01×10⁵ Pa FED standards: class 100
Bake conditions: 130°Cx60 minutes (in dry nitrogen)

Thereafter, as a hole blocking layer, a pyridine derivative (HB-1) shown below was stacked to a thickness of 10 nm by vapor deposition at a crucible temperature of 251 to 252°C and a vapor deposition rate of 0.08 to 0.12 nm/sec. The degree of vacuum during vapor deposition was from 2.1 to 2.4×10⁻⁴ Pa (approximately from 1.6 to 1.8×10⁻⁶ Torr).

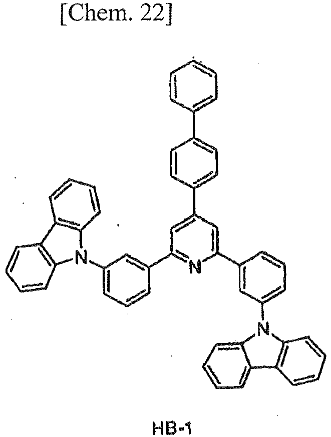

Furthermore, on the hole blocking layer, an aluminum 8-hydroxyquinoline complex (ET-1) shown below was vapor deposited as an electron transport layer in the same manner. At this time, the vapor deposition was controlled such that the crucible temperature of the aluminum 8-hydroxyquinoline complex was from 222 to 239°C, the degree of vacuum during vapor deposition was from 1.7 to 2.0×10⁻⁴ Pa (about 1.3 to 1.5×10⁻⁶ Torr), the vapor deposition rate was 0.1 nm/sec, and the film thickness was 30 nm.

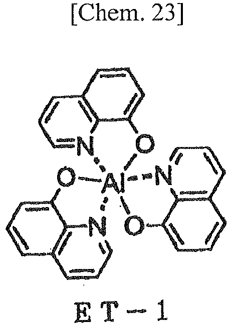

The substrate temperature when vacuum depositing the hole blocking layer and the electron transport layer was kept at room temperature.
Here, the element after completing vapor deposition up to the electron transport layer was once taken out of the vacuum deposition apparatus into the atmosphere, and a shadow mask with 2 mm-wide stripes was placed as a mask for cathode deposition to make tight contact by arranging the stripes to cross the ITO stripes of the anode 2 at right angles. The element with the mask was placed in another vacuum deposition apparatus, and similarly to the organic layer deposition, the inside of the apparatus was evacuated until the degree of vacuum was decreased to 2.3×10⁻⁶ Torr (about 3.0×10⁻⁴ Pa) or less.

Subsequently, on the electron transport layer, lithium fluoride (LiF) was deposited as an electron injection layer to a film thickness of 0.5 nm on the electron transport layer by using a molybdenum boat at a vapor deposition rate of 0.015 nm/sec and a degree of vacuum of 2.5×10⁻⁶ Torr (about 3.3×10⁻⁴ Pa).
Thereafter, aluminum was heated in a molybdenum boat and deposited as a cathode on the electron injection layer at a vapor deposition rate of 0.5 to 3.0 nm/sec and a degree of vacuum of 3.3 to 7.5×10⁻⁶ Torr (about 4.4 to 10.0×10⁻⁴ Pa) to form an aluminum layer having a film thickness of 80 nm, thereby completing the cathode.
During vapor deposition of these electron injection layer and cathode, the substrate temperature was kept at room temperature.
In this way, an organic electroluminescence element having a light-emitting area portion with a size of 2 mmx2 mm was obtained.
The maximum wavelength in the luminescence spectrum of the element was 465 nm and was identified to be derived from the fluorescent dopant (DO-1). The chromaticity was: CIE (x, y)=(0.142, 0.167).

### (Example 2)

An organic electroluminescence element was manufactured in the same manner as in Example 1 except that a compound (HO-2) having a structural formula shown below was used as the material of the luminescent layer in place of the organic compound (HO-1) and the composition was spin-coated under the same conditions.
By the spin coating, a uniform thin film (luminescent layer) having a thickness of 40 nm was formed.

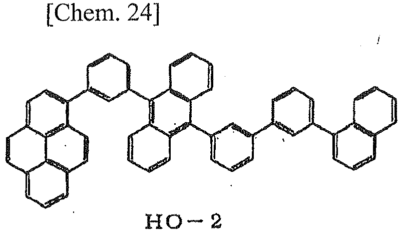

### (Reference Example 1)

An organic electroluminescence element was manufactured in the same manner as in Example 1 except that an organic compound (HO-3) having a structural formula shown below was used as the material of the luminescent layer in place of the organic compound (HO-1) and the composition was spin-coated under the same conditions.
By the spin coating, a uniform thin film (luminescent layer) of 40 nm was formed.

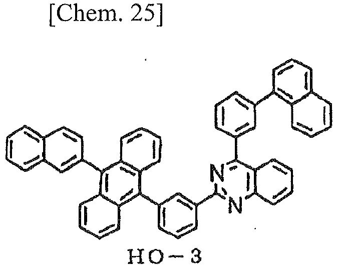

### (Comparative Example 1)

An organic electroluminescence element was manufactured in the same manner as in Example 1 except that as the film deposition conditions of the luminescent layer, the spin coating environment was changed from an oxygen concentration of 20 vol% to a nitrogen gas (oxygen concentration: 2 ppm or less).

### (Comparative Example 2)

An organic electroluminescence element was manufactured in the same manner as in Example 2 except that as the film deposition conditions of the luminescent layer, the spin coating environment was changed from an oxygen concentration of 20 vol% to a nitrogen gas (oxygen concentration: 2 ppm or less).

### (Comparative Example 3)

An organic electroluminescence element was manufactured in the same manner as in Reference Example 1 except that as the film deposition conditions of the luminescent layer, the spin coating environment was changed from an oxygen concentration of 20 vol% to a nitrogen gas (oxygen concentration: 2 ppm or less).

The characteristics of the organic electroluminescent elements produced in Examples 1 and 2, Reference Example 1 and Comparative Examples 1 to 3 are shown together in Table 1, and the time until the luminance was decreased to 500 cd/m² in a direct-current driving test with an initial luminance of 1,000 cd/m² (period of luminance decay by 50%) is shown in Table 2.

**[Table 1]**

| | Charge Transport Material | Luminance/Current [cd/A] | Voltage [V] | Luminous Efficiency [lm/W] |
|---|---|---|---|---|
| Example 1 | HO-1 | 4.0 | 8.5 | 1.8 |
| Example 2 | HO-2 | 4.1 | 8.9 | 2.0 |
| Reference Example 1 | HO-3 | 1.8 | 10.0 | 0.8 |
| Comparative Example 1 | HO-1 | 2.2 | 9.2 | 1.1 |
| Comparative Example 2 | HO-2 | 4.1 | 8.2 | 1.8 |
| Comparative Example 3 | HO-3 | 2.0 | 9.4 | 0.9 |

**[Table 2]**

| | Charge Transport Material | Oxygen Concentration at Coating | Half Life Normalized to Comparative Example 1 Taken as 1 | Ratio of Normalized Half Lives |
|---|---|---|---|---|
| Example 1 | HO-1 | 20 vol% | 31.5 | 31.5 |
| Comparative Example 1 | | ≤2 ppm | 1.0 | |
| Example 2 | HO-2 | 20 vol% | 17.0 | 17.0 |
| Comparative Example 2 | | ≤2 ppm | 1.0 | |
| Reference Example 1 | HO-3 | 20 vol% | 1.6 | 1.2 |
| Comparative Example 3 | | ≤2 ppm | 1.4 | |

As seen in Table 2, the element manufactured by the manufacturing method of an organic electroluminescence element of the present invention has a long drive life.

### (Example 4)

An organic electroluminescence element was manufactured in the same manner as in Example 1 except that in Example 1, the methods for forming the hole injection layer, hole transport layer and luminescent layer were changed as follows.

### {Manufacture of ITO Substrate}

The substrate was changed to a substrate obtained by depositing an indium/tin oxide (ITO) transparent electrically conductive film to a thickness of 70 nm on a glass-made substrate.

### {Hole Injection Layer}

The hole injection layer was formed in the same manner as in Example 1 except that in the formation of the hole injection layer of Example 1, the spinner rotation in the film deposition conditions was changed to 1,500 rpm from 2,250 rpm. A uniform thin film having a thickness of 40 nm was formed.

### {Hole Transport Layer}

The hole transport layer was formed in the same manner as in Example 1 except that in the formation of the hole transport layer of Example 1, the composition for hole transport layer and the film deposition conditions were changed as follows.

### <Composition for Hole Transport Layer>

| | |
|---|---|
| Solvent: xylene | |
| Coating solution concentration: | PB-2: 1.0 wt% |

### <Film Deposition Conditions>

Spinner rotation speed: 3,500 rpm
Spinner rotation time: 50 seconds
Spin coating atmosphere: in the atmosphere
Bake conditions: 230×60 minutes (in dry nitrogen)
By the spin coating above, a uniform thin film having a thickness of 20 nm was formed.

### {Luminescent layer}

Next, in forming a luminescent layer, an organic electroluminescence element composition shown below was prepared by using a compound (HO-4) shown below as the charge transport material and a compound (DO-2) shown below as the luminescent material and spin-coated on the hole transport layer under the following conditions to obtain a luminescent layer having a film thickness of 50 nm.

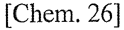

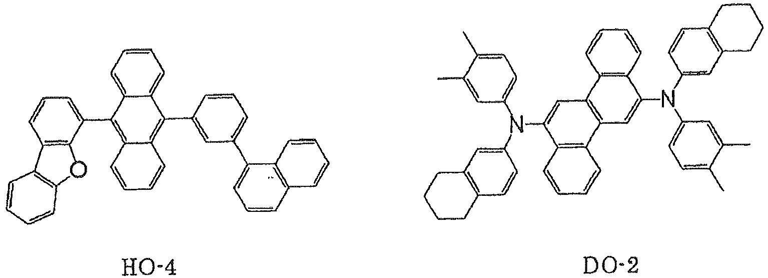

### <Luminescent layer-Forming Composition>

| | |
|---|---|
| Solvent: xylene | |
| Coating solution concentration: | HO-4: 2.0 wt% |
| | DO-2: 0.2 wt% |

### <Film Deposition Conditions>

Spinner rotation speed: 2,600 rpm
Spinner rotation time: 50 seconds
Spin coating atmosphere:
oxygen concentration of 20%, 25°C, 1.01×10⁵ Pa FED standards: class 100
Bake conditions: 130×60 minutes (in dry nitrogen)

### (Comparative Example 4)

An organic electroluminescence element was manufactured in the same manner as in Example 4 except that in Example 4, as the film deposition conditions of the hole transport layer and luminescent layer, the spin coating environment was changed to a nitrogen gas (oxygen concentration: 2 ppm or less).

The characteristics of the organic electroluminescent elements produced in Example 4 and Comparative Example 4 are shown together in Table 3, and the time until the luminance was decreased to 500 cd/m² in a direct-current driving test with an initial luminance of 1,000 cd/m² (period of luminance decay by 50%) was shown in Table 4.

**[Table 3]**

| | Charge Transport Material | Luminance/Current [cd/A] | Voltage [V] | Luminous Efficiency [lm/W] |
|---|---|---|---|---|
| Example 4 | HO-4 | 5.7 | 8.3 | 2.1 |
| Comparative Example 4 | | 5.1 | 8.0 | 2.0 |

**[Table 4]**

| | Charge Transport Material | Oxygen Concentration at Coating | Half Life Normalized to Comparative Example 1 Taken as 1 | Ratio of Normalized Half Lives |
|---|---|---|---|---|
| Example 4 | HO-4 | 20 vol% | 86.2 | 2.2 |
| Comparative Example 4 | | ≤2 ppm | 39.5 | |

As seen in Table 4, the organic electroluminescence element of the present invention has a long drive life.

### (Reference Example 2)

PB-3 shown below was used as the material of the hole injection layer in place of PB-1 used in Example 1, and the composition was spin-coated under the following conditions.

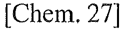

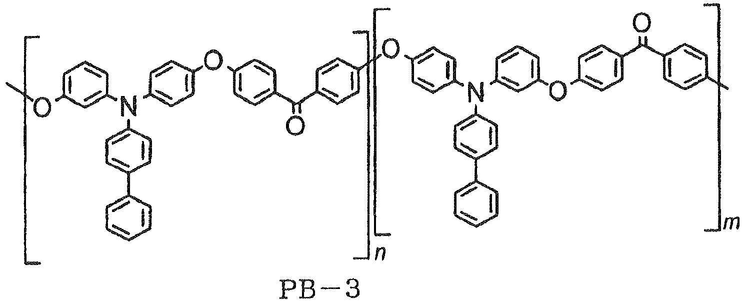

### <Composition for Hole Injection Layer>

| | |
|---|---|
| Solvent: ethyl benzoate | |
| Coating solution concentration: | PB-3: 2.0 wt% |
| | A-2: 0.8 wt% |

### <Film Deposition Conditions>

Spinner rotation speed: 2,000 rpm
Spinner rotation time: 30 seconds
Spin coating atmosphere: in the atmosphere
Bake conditions: 230°Cx30 minutes (in the atmosphere)
By the spin coating above, a uniform thin film having a thickness of 30 nm was formed.

Without providing a hole transport layer, as the material of the luminescent layer, organic compounds (HO-5) and (HO-6) shown below and luminescent materials (DO-3) and (DO-4) were used in place of the organic compound (HO-1) and the luminescent material (DO-1) used in Example 1, and the composition was spin-coated under the following conditions.

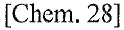

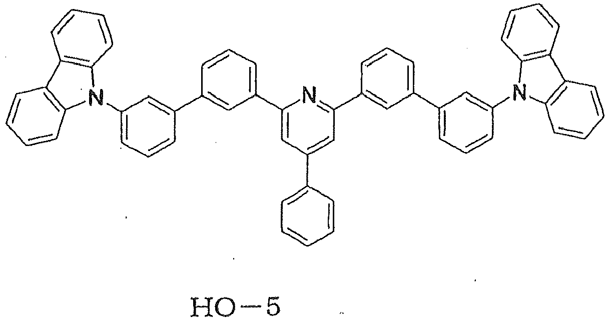

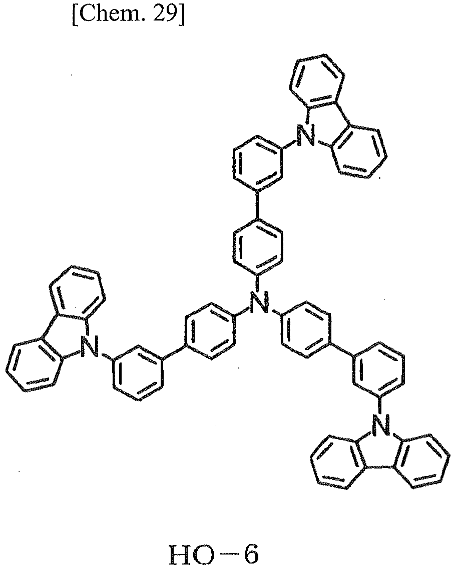

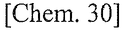

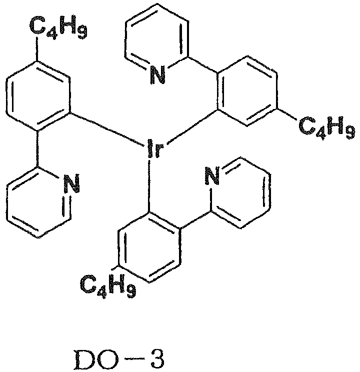

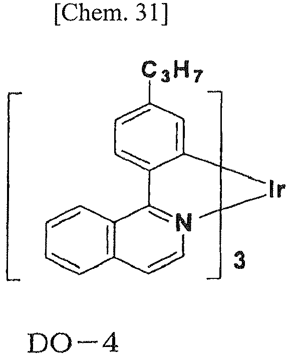

### <Luminescent layer-Forming Composition>

| | |
|---|---|
| Solvent: xylene | |
| Concentration in composition: | HO-5: 1.3 wt% |
| | HO-6: 1.3 wt% |
| | DO-3: 0.1 wt% |
| | DO-4:0.1 wt% |

### <Film Deposition Conditions>

Spinner rotation speed: 1,500 rpm
Spinner rotation time: 30 seconds
Spin coating atmosphere: oxygen concentration of 20%, 25°C, 1.01×10⁵ Pa FED standards: class 100
Bake conditions: 130°C×60 minutes (in dry nitrogen)

An organic electroluminescence element was manufactured in the same manner as in Example 1 except that as the hole blocking layer, (ET-2) shown below was used in place of the pyridine derivative (HB-1) and the film thickness was changed to 5 nm.

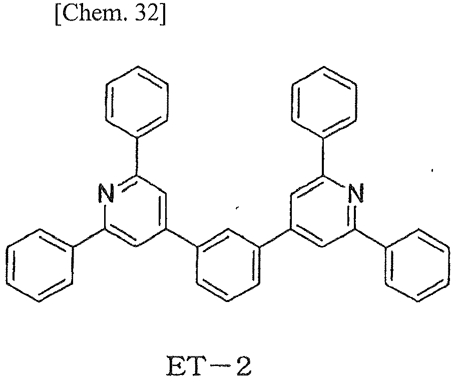

### (Reference Example 3)

An organic electroluminescence element was manufactured in the same manner as in Reference Example 2 except that as the film deposition conditions of the luminescent layer, the spin coating environment was changed from an oxygen concentration of 20 vol% to a nitrogen gas (oxygen concentration: 2 ppm or less).

**[Table 5]**

| | Charge Transport Material | Luminance/Current [cd/A] | Voltage [V] | Efficiency [lm/W] |
|---|---|---|---|---|
| Reference Example 2 | HO-5 | 3.2 | 9.2 | 1.1 |
| Reference Example 3 | HO-6 | 4.1 | 11.1 | 1.2 |

**[Table 6]**

| | Charge Transport Material | Oxygen Concentration at Coating | Half Life Normalized to Comparative Example 1 Taken as 1 | Ratio of Normalized Half Lives |
|---|---|---|---|---|
| Reference Example 2 | HO-5 | 20 vol% | 69.8 | 0.54 |
| Reference Example 3 | HO-6 | ≤2 ppm | 128.9 | |

### (Comparative Example 5)

Manufacture of an organic electroluminescence element was tried in the same manner as in Example 1 by using, as the material used in the luminescent layer, 9,10-diphenylanthracene (HO-7) shown below in place of the organic compound (HO-1), but there was caused a problem that a crystal was generated in the coating formed using HO-7 and the film was whitened. That is, HO-7 was not suited for the method manufacturing for an organic electroluminescence element of the present invention, where the luminescent layer is formed by a wet film-forming method.

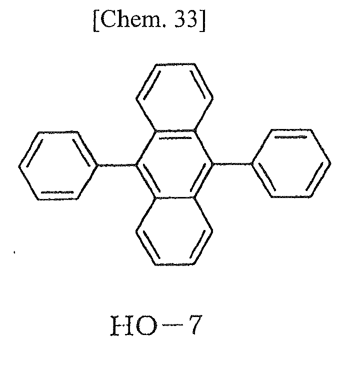

While the invention has been described in detail and with reference to specific embodiments thereof, it will be apparent to one skilled in the art that various changes and modifications can be made therein without departing from the spirit and scope of the invention. This application is based on Japanese Patent Application (Patent Application No. 2009-284384) filed on December 15, 2009 and Japanese Patent Application (Patent Application No. 2010-16030) filed on January 27, 2010, the contents of which are incorporated herein by way of reference.

### INDUSTRIAL APPLICABILITY

The organic electroluminescence element manufactured by the manufacturing method of the present invention is suited for a display device or a lighting device and, specifically, is thought to be applicable to flat panels/displays (for example, an office automation (OA) computer display and a thin television), vehicle-mounted display elements, cellular phone displays, light sources taking advantage of the feature of a surface light emitter (for example, a light source of copiers and a backlight source of liquid crystal displays or instruments), display boards, and marker lights, and its technical value is very high.

### DESCRIPTION OF REFERENCE NUMERALS AND SIGNS

- 1: Substrate
- 2: Anode
- 3: Hole injection layer
- 4: Hole transport layer
- 5: Luminescent layer
- 6: Hole blocking layer
- 7: Electron transport layer
- 8: Electron injection layer
- 9: Cathode

## Claims

1. A method for manufacturing an organic electroluminescence element having a luminescent layer between a first electrode and a second electrode formed to face said first electrode, the method comprising,
as a luminescent layer-forming step, forming a coating film by a wet film-forming method in an environment with an oxygen concentration of 18 to 22 vol% by using a luminescent layer-forming composition containing: a compound having a molecular weight of 400 or more and represented by the following formula (1); and a solvent: 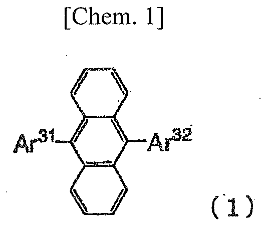 (wherein each of Ar³¹ and Ar³² independently represents an aromatic hydrocarbon ring group or aromatic heterocyclic group having a substituent, provided that said compound represented by formula (1) does not have a partial structure derived from a nitrogen atom-containing heterocyclic ring, and
the anthracene ring in formula (1) may further have an optional substituent).

2. The method of manufacturing an organic electroluminescence element as claimed in claim 1, which further comprises heating said coating film in an inert gas.

3. The method of manufacturing an organic electroluminescence element as claimed in claim 1 or 2, wherein the molecular weight of said compound represented by formula (1) is 10,000 or less.

4. The method of manufacturing an organic electroluminescence element as claimed in any one of claims 1 to 3, wherein said compound represented by formula (1) is a charge transport material.

5. The method of manufacturing an organic electroluminescence element as claimed in any one of claims 1 to 4, wherein said luminescent layer-forming composition further contains a luminescent material and said luminescent material contains a condensed aromatic hydrocarbon ring having a nuclear carbon number of 5 to 40, which may be substituted.

6. The method of manufacturing an organic electroluminescence element as claimed in claim 5, wherein said luminescent material contains at least one compound selected from the group consisting of naphthalene, perylene, pyrene, chrysene, anthracene, coumarin, p-bis(2-phenylethenyl)benzene, a styrylamine compound represented by the following formula (2), and an arylamine compound represented by the following formula (3): 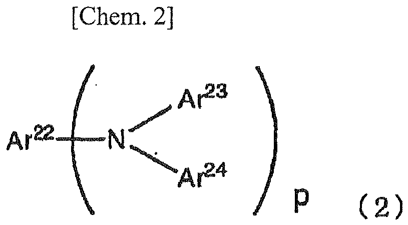 (wherein Ar²² represents a group derived from an aromatic hydrocarbon ring or an aromatic heterocyclic ring, or a stilbene-derived group, each of which may be substituted,
each of Ar²³ and Ar²⁴ independently represents a hydrogen atom, a styryl group which may be substituted, or an aromatic hydrocarbon ring group having a carbon number of 6 to 20, which may be substituted, and
p represents an integer of 1 to 4,
provided that when Ar²² is not a stilbene-derived group and at the same time, the group derived from an aromatic hydrocarbon ring or an aromatic heterocyclic ring is not substituted with a styryl group, at least one of Ar²³ and Ar²⁴ is a styryl group which may be substituted); 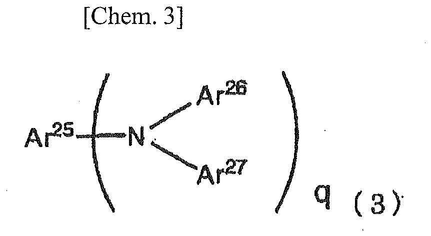 (wherein Ar²⁵ represents an aromatic hydrocarbon ring-derived group having a nuclear carbon number of 10 to 40, which may be substituted, or an aromatic heterocyclic ring-derived group which may be substituted,
each of Ar²⁶ and Ar²⁷ independently represents an aromatic hydrocarbon ring-derived group having a nuclear carbon number of 5 to 40, which may be substituted, or an aromatic heterocyclic ring-derived group which may be substituted, and
q represents an integer of 1 to 4).

7. The method of manufacturing an organic electroluminescence element as claimed in claim 5 or 6, wherein the content of said compound represented by formula (1) in said luminescent layer-forming composition is, on the weight basis, 2 times or more the entire amount of said luminescent material.

8. An organic electroluminescence element manufactured by the manufacturing method claimed in any one of claims 1 to 7.

9. A display device comprising the organic electroluminescence element claimed in claim 8.

10. A lighting device comprising the organic electroluminescence element claimed in claim 8.
